# EUROPEAN PATENT APPLICATION

(11) **EP 4 825 485 A1**
(43) Date of publication of application: **21.10.2026**
(21) Application number: 24903264.0
(22) Date of filing: 12.09.2024
(51) Int. Cl.: B01J 20/281, C07K 1/22, G01N 30/50

(54) **PRODUCTION METHOD FOR CHROMATOGRAPHIC CARRIER**

(30) Priority: 13.12.2023 JP 2023210494
(71) Applicant: JSR Corporation, Tokyo 105-8640 (JP)
(72) Inventor: MIYAJIMA, Ken, Tokyo 105-8640 (JP); AKIYAMA, Minato, Tokyo 105-8640 (JP); MATSUMOTO, Kentarou, Tokyo 105-8640 (JP); KOBAYASHI, Kunihiko, Tokyo 105-8640 (JP)
(74) Representative: TBK
(86) International application number: PCT/JP2024/032743
(87) International publication number: WO 2025/126601

(57) **Abstract**

To provide a method for easily producing a chromatographic carrier having a large dynamic binding capacity for an antibody or a fragment thereof, in which a proteinaceous ligand is less likely to leak during isolation, and aggregation between carriers is less likely to occur.

A production method for a chromatographic carrier, including the following solid-phase carrier washing process, ligand binding process, and ligand-bound carrier washing process.
(Solid-phase carrier washing process) a process of washing a solid-phase carrier with at least one washing liquid selected from the group consisting of a washing liquid containing hydrogen peroxide, a washing liquid containing peracetic acid, and a washing liquid having a pH of 0 or more and 3 or less or a pH of more than 12.5 and 14 or less (excluding a washing liquid containing hydrogen peroxide and a washing liquid containing peracetic acid), thereby hydrophilizing a surface of the solid-phase carrier
(Ligand binding process) a process of binding a proteinaceous ligand to the solid-phase carrier washed in the solid-phase carrier washing process
(Ligand-bound carrier washing process) a process of washing the carrier obtained in the ligand binding process

## Description

### Technical Field

The present invention relates to a production method for a chromatographic carrier.

### Background Art

In recent years, in the field of biopharmaceuticals represented by antibody drugs and the like, expression technologies for target substances such as proteins have remarkably advanced, and accordingly, improvement in production efficiency in purification processes such as chromatography has been required. As a method for improving productivity, it has been proposed to reduce the concentrations of impurities mixed in pharmaceutical raw materials, such as host cell-derived proteins and deoxyribonucleic acid, as much as possible by one purification, and to reduce the number of purifications and processes, and demand for a chromatographic carrier capable of realizing this has been increasing. As a production method for such a chromatographic carrier, for example, a method is known in which a synthetic polymer-based solid-phase carrier or a natural polymer-based solid-phase carrier is obtained by a polymerization reaction, a crosslinking reaction, a functional group introduction reaction, or the like, and a proteinaceous ligand is bound thereto (Patent Literature 1).

### Citation List

### Patent Literature

Patent Literature 1: JP 2017-37069 A
Patent Literature 2: WO2008/146906
Patent Literature 3: WO2019/121296

### Summary of Invention

### Technical Problem

Further, the carrier to which the proteinaceous ligand obtained as described above is bound has impurities adhering thereto, including unreacted proteinaceous ligand and other impurities generated in each production process. Therefore, for the purpose of preventing unreacted proteinaceous ligand from leaking out and being mixed into an antibody during antibody purification, the carrier to which the ligand has been bound may be subjected to stirring washing with an aqueous sodium hydroxide solution or hydrochloric acid after the ligand is bound (Patent Literature 2 and Patent Literature 3). However, the ligand may be damaged by sodium hydroxide or hydrochloric acid, and the dynamic binding capacity for an antibody or a fragment thereof may decrease. Further, in a case where only such washing is performed, aggregation between carriers tends to occur. Carriers in which aggregation between carriers is likely to occur may cause variation in performance between lots.

A problem to be solved by the present invention is to provide a method for easily producing a chromatographic carrier having a large dynamic binding capacity for an antibody or a fragment thereof, in which a proteinaceous ligand is less likely to leak during isolation, and aggregation between carriers is less likely to occur.

### Solution to Problem

The present inventors have found that, in addition to performing washing after binding a proteinaceous ligand to a solid-phase carrier, by washing the solid-phase carrier, prior to the binding of the proteinaceous ligand, with a specific washing liquid that is at least one washing liquid selected from the group consisting of a washing liquid containing hydrogen peroxide, a washing liquid containing peracetic acid, and a washing liquid having a pH of 0 or more and 3 or less or a pH of more than 12.5 and 14 or less (excluding a washing liquid containing hydrogen peroxide and a washing liquid containing peracetic acid), the surface of the solid-phase carrier is hydrophilized, the dynamic binding capacity of the chromatographic carrier for an antibody or a fragment thereof increases and aggregation between carriers is less likely to occur, and, despite the above washing being performed prior to the binding of the proteinaceous ligand, the proteinaceous ligand is less likely to leak during isolation.

That is, the present invention provides the following <1> to <16>.
<1> A production method for a chromatographic carrier, comprising the following solid-phase carrier washing process, ligand binding process, and ligand-bound carrier washing process (hereinafter also referred to as the production method for a chromatographic carrier of the present invention).
   (Solid-phase carrier washing process) a process of washing a solid-phase carrier with at least one washing liquid selected from the group consisting of a washing liquid containing hydrogen peroxide, a washing liquid containing peracetic acid, and a washing liquid having a pH of 0 or more and 3 or less or a pH of more than 12.5 and 14 or less (excluding a washing liquid containing hydrogen peroxide and a washing liquid containing peracetic acid), thereby hydrophilizing a surface of the solid-phase carrier
   (Ligand binding process) a process of binding a proteinaceous ligand to the solid-phase carrier washed in the solid-phase carrier washing process
   (Ligand-bound carrier washing process) a process of washing the carrier obtained in the ligand binding process
<2> The production method for a chromatographic carrier according to <1>, in which the ligand-bound carrier washing process is a process of washing with a washing liquid having a pH of more than 3 and 12.5 or less.
<3> The production method for a chromatographic carrier according to <2>, in which the ligand-bound carrier washing process is a process of washing the carrier obtained in the ligand binding process two or more times in total with a washing liquid having a pH of more than 3 and 12.5 or less.
<4> The production method for a chromatographic carrier according to <2>, in which the ligand-bound carrier washing process is a process of washing the carrier obtained in the ligand binding process 3 or 4 times in total with a washing liquid having a pH of more than 3 and 12.5 or less.
<5> The production method for a chromatographic carrier according to any one of <1> to <4>, in which the ligand-bound carrier washing process is a process including the following ligand-bound carrier bed formation process, ligand-bound carrier liquid-passing washing process, and ligand-bound carrier stirring washing process.
   (Ligand-bound carrier bed formation process) a process of filling a ligand-bound carrier into a container to form a ligand-bound carrier bed
   (Ligand-bound carrier liquid-passing washing process) a process of washing the ligand-bound carrier bed formed in the ligand-bound carrier bed formation process by passing a washing liquid having a pH of more than 3 and 12.5 or less therethrough one or more times
   (Ligand-bound carrier stirring washing process) a process of washing the ligand-bound carrier after the ligand-bound carrier liquid-passing washing process by stirring in a washing liquid having a pH of more than 3 and 12.5 or less one or more times
<6> The production method for a chromatographic carrier according to <5>, in which the number of times of liquid-passing washing in the ligand-bound carrier liquid-passing washing process is 2 to 5.
<7> The production method for a chromatographic carrier according to any one of <1> to <6>, in which the solid-phase carrier washing process is a process of hydrophilizing a surface of the solid-phase carrier, the process including the following solid-phase carrier bed formation process and solid-phase carrier liquid-passing washing process.
   (Solid-phase carrier bed formation process) a process of filling a solid-phase carrier into a container to form a solid-phase carrier bed
   (Solid-phase carrier liquid-passing washing process) a process of washing the solid-phase carrier bed formed in the solid-phase carrier bed formation process by passing at least one washing liquid selected from the group consisting of a washing liquid containing hydrogen peroxide, a washing liquid containing peracetic acid, and a washing liquid having a pH of 0 or more to 3 or less or a pH of more than 12.5 and 14 or less (excluding a washing liquid containing hydrogen peroxide and a washing liquid containing peracetic acid) therethrough one or more times
<8> The production method for a chromatographic carrier according to any one of <1> to <6>, in which the solid-phase carrier washing process is a process of hydrophilizing a surface of the solid-phase carrier, the process including the following solid-phase carrier bed formation process, solid-phase carrier liquid-passing washing process, and solid-phase carrier stirring washing process.
   (Solid-phase carrier bed formation process) a process of filling a solid-phase carrier into a container to form a solid-phase carrier bed
   (Solid-phase carrier liquid-passing washing process) a process of washing the solid-phase carrier bed formed in the solid-phase carrier bed formation process by passing at least one washing liquid selected from the group consisting of a washing liquid containing hydrogen peroxide, a washing liquid containing peracetic acid, and a washing liquid having a pH of 0 or more to 3 or less or a pH of more than 12.5 and 14 or less (excluding a washing liquid containing hydrogen peroxide and a washing liquid containing peracetic acid) therethrough one or more times
   (Solid-phase carrier stirring washing process) a process of washing the solid-phase carrier after the solid-phase carrier liquid-passing washing process by stirring in at least one washing liquid selected from the group consisting of a washing liquid containing hydrogen peroxide, a washing liquid containing peracetic acid, and a washing liquid having a pH of 0 or more and 3 or less or a pH of more than 12.5 and 14 or less (excluding a washing liquid containing hydrogen peroxide and a washing liquid containing peracetic acid) one or more times
<9> The production method for a chromatographic carrier according to <7> or <8>, in which the number of times of liquid-passing washing in the solid-phase carrier liquid-passing washing process is 2 to 5.
<10> The production method for a chromatographic carrier according to <7> or <8>, in which the sum of the number of times of liquid-passing washing of the solid-phase carrier liquid-passing washing process and the number of times of liquid-passing washing of the ligand-bound carrier liquid-passing washing process is 2 to 8.
<11> The production method for a chromatographic carrier according to any one of <1> to <10>, in which the ligand-bound carrier washing process is a process of washing with a washing liquid having a pH of 10.5 or more and 12 or less.
<12> The production method for a chromatographic carrier according to any one of <1> to <11>, in which the washing liquid used in the solid-phase carrier washing process contains a strongly acidic pH adjusting agent or a strongly basic pH adjusting agent, and the washing liquid used in the ligand-bound carrier washing process contains an acidic pH adjusting agent or a basic pH adjusting agent.
<13> The production method for a chromatographic carrier according to any one of <1> to <12>, in which the washing liquid used in the solid-phase carrier washing process is a washing liquid having a pH of 13.2 or more and 14 or less (excluding a washing liquid containing hydrogen peroxide and a washing liquid containing peracetic acid).
<14> The production method for a chromatographic carrier according to any one of <1> to <13>, in which the solid-phase carrier washing process is a process of washing a solid-phase carrier two or more times in total with at least one washing liquid selected from the group consisting of a washing liquid containing hydrogen peroxide, a washing liquid containing peracetic acid, and a washing liquid having a pH of 0 or more and 3 or less or a pH of more than 12.5 and 14 or less (excluding a washing liquid containing hydrogen peroxide and a washing liquid containing peracetic acid).
<15> The production method for a chromatographic carrier according to any one of <1> to <14>, in which the solid-phase carrier washing process is a process of washing a solid-phase carrier 3 to 4 times in total with at least one washing liquid selected from the group consisting of a washing liquid containing hydrogen peroxide, a washing liquid containing peracetic acid, and a washing liquid having a pH of 0 or more and 3 or less or a pH of more than 12.5 and 14 or less (excluding a washing liquid containing hydrogen peroxide and a washing liquid containing peracetic acid).
<16> The production method for a chromatographic carrier according to any one of <1> to <15>, in which the proteinaceous ligand is a modified protein A.

### Advantageous Effects of Invention

According to the production method for a chromatographic carrier of the present invention, a chromatographic carrier having a large dynamic binding capacity for an antibody or a fragment thereof, in which a proteinaceous ligand is less likely to leak during isolation, and in which aggregation between carriers is less likely to occur can be easily produced.

### Description of Embodiments

### [Method for Producing Chromatographic Carrier]

The production method for a chromatographic carrier of the present invention comprises the following solid-phase carrier washing process, ligand binding process, and ligand-bound carrier washing process.
(Solid-phase carrier washing process) a process of washing a solid-phase carrier with at least one washing liquid selected from the group consisting of a washing liquid containing hydrogen peroxide, a washing liquid containing peracetic acid, and a washing liquid having a pH of 0 or more and 3 or less or a pH of more than 12.5 and 14 or less (excluding a washing liquid containing hydrogen peroxide and a washing liquid containing peracetic acid), thereby hydrophilizing a surface of the solid-phase carrier
(Ligand binding process) a process of binding a proteinaceous ligand to the solid-phase carrier washed in the solid-phase carrier washing process
(Ligand-bound carrier washing process) a process of washing the carrier obtained in the ligand binding process

### (Solid-phase Carrier Washing Process)

A solid-phase carrier washing process is a process of washing a solid-phase carrier with at least one washing liquid selected from the group consisting of a washing liquid containing hydrogen peroxide, a washing liquid containing peracetic acid, and a washing liquid having a pH of 0 or more and 3 or less or a pH of more than 12.5 and 14 or less (excluding a washing liquid containing hydrogen peroxide and a washing liquid containing peracetic acid), thereby hydrophilizing a surface of the solid-phase carrier This process is performed prior to the binding of the proteinaceous ligand.

By providing such a solid-phase carrier washing process, a chromatographic carrier having a large dynamic binding capacity for an antibody or a fragment thereof, in which a proteinaceous ligand is less likely to leak during isolation, and in which aggregation between carriers is less likely to occur is obtained. Further, it becomes possible to use washing liquids having various pH values as the washing liquid used in the ligand-bound carrier washing process. For example, even in a case where a washing liquid having a pH of more than 3 and 12.5 or less is used in the ligand-bound carrier washing process, a chromatographic carrier in which a proteinaceous ligand is less likely to leak during isolation is obtained. Although the reason why the ligand is less likely to leak during isolation in this manner is not necessarily clear, the present inventors presume that the surface of the solid-phase carrier is hydrophilized by the solid-phase carrier washing process to have excellent antifouling properties, and that unreacted proteinaceous ligand that generally adheres in the subsequent ligand binding process can be more readily washed even when the pH of the washing liquid is, for example, more than 3 and 12.5 or less. For example, in a case where the solid-phase carrier contains, in a molecule thereof, a functional group capable of binding a ligand such as a cyclic ether group or a hydroxyl group, or a functional group such as a carbonyl bond, the present inventors presume that hydrophilicity is increased and excellent antifouling properties are provided because these functional groups are hydrolyzed or because a large amount of hydroxyl groups or carboxyl groups is present.

Further, the production method for a chromatographic carrier of the present invention is such that the solid-phase carrier is washed prior to the binding of the ligand; nevertheless, a ligand is sufficiently bound to the solid-phase carrier in the ligand binding process, and a chromatographic carrier having a large dynamic binding capacity for an antibody or a fragment thereof can be produced. Although the reason why such dynamic binding capacity is obtained is not necessarily clear, the present inventors presume that one reason is that damage to the chromatographic carrier can be significantly reduced by appropriately combining a plurality of washing processes at appropriate timings.

In the present invention, "hydrophilization" means that affinity for water becomes higher.

As the solid-phase carrier, a solid-phase carrier having, in a molecule thereof, a functional group capable of binding a ligand (for example, a functional group selected from the group consisting of a cyclic ether group, a carboxyl group, -C(=O)-O-C(=O)-, a succinimide oxycarbonyl group, a formyl group, a hydroxyl group, and an isocyanate group) is preferred. In a case where such a solid-phase carrier is used, particularly excellent low-leakage properties of a proteinaceous ligand tend to be obtained.

Examples of the solid-phase carrier include a particulate solid-phase carrier, a monolithic solid-phase carrier, a plate-shaped solid-phase carrier, a membrane-shaped solid-phase carrier, a fibrous solid-phase carrier, and a chip-shaped solid-phase carrier, among which a particulate solid-phase carrier is preferred, and a porous particulate solid-phase carrier (hereinafter also referred to simply as "porous particles") is more preferred.

As the porous particles, porous particles including a polymer are preferred. Such porous particles may be natural polymer-based porous particles composed of polysaccharides such as agarose, dextran, and cellulose, or synthetic polymer-based porous particles. In order to increase dynamic binding capacity and to improve uniformity of particle diameter, synthetic polymer-based porous particles are preferred. In addition, the porous particles are preferably water-insoluble.

As the solid-phase carrier, a commercially available product may be used, or one produced according to a conventional method may be used. Here, a production method for the solid-phase carrier will be described.

In a case where porous particles are obtained as the solid-phase carrier, the porous particles can be produced by a method including a process of dispersing a monomer composition in an aqueous medium and subjecting the monomer composition to suspension polymerization (hereinafter also referred to as Process P1).

### -Process P1-

As the monomer composition used in Process P1, a composition containing a functional group-containing monomer is preferred. The functional group contained in this monomer is preferably one that can be used for an additional chemical reaction (such as a reaction with a crosslinking agent), and may be one capable of binding a ligand. Examples of the functional group include a functional group selected from the group consisting of a cyclic ether group, a carboxyl group, -C(=O)-O-C(=O)-, a succinimide oxycarbonyl group, a formyl group, a hydroxyl group, and an isocyanate group. Among these, a cyclic ether group is preferred.

Here, as the "cyclic ether group," a cyclic ether group having 3 to 7 atoms constituting the ring is preferred. The cyclic ether group may have an alkyl group as a substituent. Specific examples of the cyclic ether group include cyclic ether groups represented by Formulae (4) to (9) below, among which cyclic ether groups represented by Formulae (4), (6), or (9) are preferred, and cyclic ether groups represented by Formula (4) are more preferred.

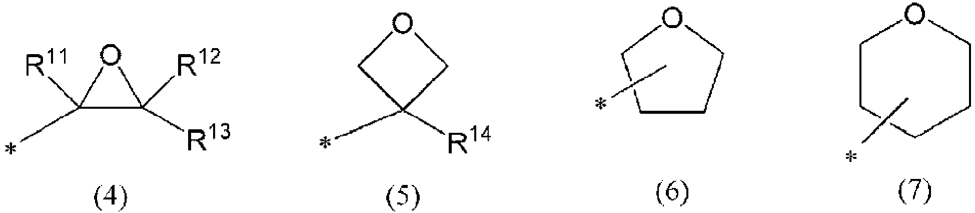

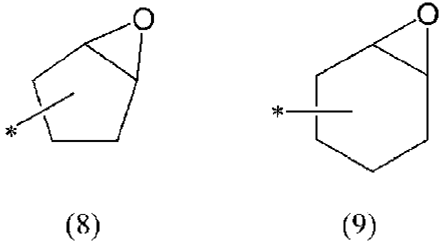

[In the formula, R¹¹ to R¹⁴ each independently represent a hydrogen atom or an alkyl group, and * represents a bonding site.]

The alkyl group represented by R¹¹ to R¹⁴ preferably has 1 to 4 carbon atoms, and more preferably 1 or 2 carbon atoms. The alkyl group may be linear or branched, and examples thereof include a methyl group, an ethyl group, an n-propyl group, an isopropyl group, an n-butyl group, a sec-butyl group, and a tert-butyl group. In addition, as R¹¹ to R¹⁴, a hydrogen atom is preferred.

As the functional group-containing monomer, a monomer having a functional group capable of binding a ligand and a polymerizable unsaturated group is preferred. Examples of such a monomer include (meth)acrylate-based monomers having a cyclic ether group, such as glycidyl (meth)acrylate, 3-oxiranylpropyl (meth)acrylate, 4-oxiranylbutyl (meth)acrylate, 5-oxiranylpentyl (meth)acrylate, 6-oxiranylhexyl (meth)acrylate, 7-oxiranylheptyl (meth)acrylate, 8-oxiranyloctyl (meth)acrylate, (3-methyloxiranyl)methyl (meth)acrylate, 4-hydroxybutyl (meth)acrylate glycidyl ether, glycerin mono(meth)acrylate glycidyl ether, 3,4-epoxycyclohexylmethyl (meth)acrylate, 3,4-epoxycyclohexylethyl (meth)acrylate, 3,4-epoxycyclohexylpropyl (meth)acrylate, α-(meth)acryl-ω-glycidyl polyethylene glycol, and tetrahydrofurfuryl (meth)acrylate; aromatic vinyl-based monomers having a cyclic ether group, such as (vinylbenzyl) glycidyl ether, (isopropenylbenzyl) glycidyl ether, (vinylphenethyl) glycidyl ether, (vinylphenylbutyl) glycidyl ether, (vinylbenzyloxyethyl) glycidyl ether, (vinylphenyl) glycidyl ether, (isopropenylphenyl) glycidyl ether, and 1,2-epoxy-3-(4-vinylbenzyl)propane; allyl ether-based monomers having a cyclic ether group, such as allyl glycidyl ether; (meth)acrylate-based monomers having an isocyanate group, such as isocyanatoethyl (meth)acrylate; unsaturated dicarboxylic anhydride-based monomers, such as maleic anhydride, methylmaleic anhydride, and glutaconic anhydride; and (meth)acrylic acid, 3,4-epoxy-1-butene, and 3,4-epoxy-3-methyl-1-butene. These monomers may be used alone or in combinations of two or more.

Among these monomers, (meth)acrylate-based monomers having a cyclic ether group are preferred, and glycidyl (meth)acrylate is particularly preferred.

The total amount of the functional group-containing monomer used is preferably 35 parts by mass or more, more preferably 45 parts by mass or more, and particularly preferably 55 parts by mass or more, and is also preferably 99 parts by mass or less, more preferably 90 parts by mass or less, and particularly preferably 85 parts by mass or less, relative to 100 parts by mass of the total amount of monomers used in Process P1.

Further, the monomer composition used in Process P1 may further contain a monomer other than the above-described functional group-containing monomer (hereinafter also referred to as "other monomer").

Examples of the other monomer include a polymerizable unsaturated group-containing monomer that does not have a functional group capable of binding a ligand. The other monomer is broadly classified into a non-crosslinking monomer and a crosslinking monomer, and either one of these may be used alone or both may be used in combination.

Examples of the above non-crosslinking monomer include (meth)acrylate-based non-crosslinking monomers, (meth)acrylamide-based non-crosslinking monomers, aromatic vinyl-based non-crosslinking monomers, vinyl ketone-based non-crosslinking monomers, (meth)acrylonitrile-based non-crosslinking monomers, and N-vinylamide-based non-crosslinking monomers. These may be used alone or in combinations of two or more. Among these non-crosslinking monomers, (meth)acrylate-based non-crosslinking monomers and aromatic vinyl-based non-crosslinking monomers are preferred.

Examples of the above (meth)acrylate-based non-crosslinking monomers include methyl (meth)acrylate, ethyl (meth)acrylate, n-butyl (meth)acrylate, 4-tert-butyl (meth)acrylate, isobutyl (meth)acrylate, n-octyl (meth)acrylate, 2-ethylhexyl (meth)acrylate, cyclohexyl (meth)acrylate, methoxyethyl (meth)acrylate, hydroxyethyl (meth)acrylate, hydroxypropyl (meth)acrylate, glycerol mono(meth)acrylate, trimethylolethane mono(meth)acrylate, trimethylolpropane mono(meth)acrylate, butanetriol mono(meth)acrylate, polyethylene glycol mono(meth)acrylate, methoxypolyethylene glycol (meth)acrylate, pentaerythritol mono(meth)acrylate, dipentaerythritol mono(meth)acrylate, and inositol mono(meth)acrylate. These may be used alone or in combinations of two or more.

Examples of the above (meth)acrylamide-based non-crosslinking monomers include (meth)acrylamide, dimethyl (meth)acrylamide, hydroxyethyl (meth)acrylamide, (meth)acryloyl morpholine, and diacetone (meth)acrylamide. These may be used alone or in combinations of two or more.

Examples of the above aromatic vinyl-based non-crosslinking monomers include styrenes such as styrene, α-methylstyrene, halogenated styrene, 4-methylstyrene, 2,4-dimethylstyrene, 2,4,6-trimethylstyrene, ethylvinylbenzene, 4-isopropylstyrene, 4-n-butylstyrene, 4-isobutylstyrene, and 4-tert-butylstyrene; and vinylnaphthalenes such as 1-vinylnaphthalene and 2-vinylnaphthalene. These may be used alone or in combinations of two or more.

Examples of the above vinyl ketone-based non-crosslinking monomer include ethyl vinyl ketone, propyl vinyl ketone, and isopropyl vinyl ketone. These may be used alone or in combinations of two or more.

Examples of the above (meth)acrylonitrile-based non-crosslinking monomer include acrylonitrile and methacrylonitrile. These may be used alone or in combinations of two or more.

Examples of the above N-vinylamide-based non-crosslinking monomer include N-vinylacetamide and N-vinylpropionamide. These may be used alone or in combinations of two or more.

The total amount of the non-crosslinking monomer used is preferably 0.01 parts by mass or more, more preferably 0.05 parts by mass or more, and particularly preferably 0.1 parts by mass or more, and is also preferably 30 parts by mass or less, more preferably 15 parts by mass or less, and particularly preferably 5 parts by mass or less, relative to 100 parts by mass of the total amount of monomers used in Process P1.

Examples of the above crosslinking monomer include (meth)acrylate-based crosslinking monomers, aromatic vinyl-based crosslinking monomers, and allyl-based crosslinking monomers. These may be used alone or in combinations of two or more. As the crosslinking monomer, a bifunctional to pentafunctional crosslinking monomer is preferred, and a bifunctional or trifunctional crosslinking monomer is more preferred. Among these crosslinking monomers, (meth)acrylate-based crosslinking monomers and aromatic vinyl-based crosslinking monomers are preferred.

Examples of the above (meth)acrylate-based crosslinking monomer include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, tripropylene glycol di(meth)acrylate, tetrapropylene glycol di(meth)acrylate, polypropylene glycol di(meth)acrylate, 1,4-butanediol di(meth)acrylate, 1,6-hexanediol di(meth)acrylate, glycerin di(meth)acrylate, trimethylolethane di(meth)acrylate, trimethylolpropane di(meth)acrylate, trimethylolpropane tri(meth)acrylate, butanetriol di(meth)acrylate, pentaerythritol di(meth)acrylate, pentaerythritol tri(meth)acrylate, pentaerythritol tetra(meth)acrylate, glucose di(meth)acrylate, glucose tri(meth)acrylate, glucose tetra(meth)acrylate, dipentaerythritol di(meth)acrylate, dipentaerythritol tri(meth)acrylate, dipentaerythritol tetra(meth)acrylate, dipentaerythritol penta(meth)acrylate, inositol di(meth)acrylate, inositol tri(meth)acrylate, inositol tetra(meth)acrylate, mannitol di(meth)acrylate, mannitol tri(meth)acrylate, mannitol tetra(meth)acrylate, and mannitol penta(meth)acrylate. These may be used alone or in combinations of two or more.

Examples of the above aromatic vinyl-based crosslinking monomer include divinylbenzene, trivinylbenzene, divinyltoluene, divinylxylene, divinylethylbenzene, and divinylnaphthalene. These may be used alone or in combinations of two or more.

Examples of the allyl-based crosslinking monomer include diallyl phthalate, diallyl isophthalate, diallyl terephthalate, diallyl maleate, diallyl fumarate, diallyl itaconate, diallyl trimellitate, triallyl trimellitate, triallyl cyanurate, diallyl isocyanurate, and triallyl isocyanurate. These may be used alone or in combinations of two or more.

Further, as the crosslinking monomer, in addition to those exemplified above, dehydration condensation products of amino alcohols such as diaminopropanol, tris(hydroxymethyl)aminomethane, and glucosamine with (meth)acrylic acid, and conjugated diolefins such as butadiene and isoprene may be mentioned.

The total amount of the crosslinking monomer used is preferably 1 part by mass or more, more preferably 5 parts by mass or more, and particularly preferably 10 parts by mass or more, and is also preferably 50 parts by mass or less, more preferably 40 parts by mass or less, and particularly preferably 30 parts by mass or less, relative to 100 parts by mass of the total amount of monomers used in Process P1.

Examples of the aqueous medium used in Process P1 include an aqueous solution of a water-soluble polymer. Examples of the water-soluble polymer include hydroxyethyl cellulose, polyvinyl alcohol, carboxymethyl cellulose, polyvinylpyrrolidone, starch, and gelatin.

The total amount of the aqueous medium used is usually about 200 parts by mass or more and 7000 parts by mass or less relative to 100 parts by mass of the total amount of monomers.

Further, in a case where water is used as a dispersion medium of the aqueous medium, a dispersion stabilizer such as sodium carbonate, calcium carbonate, sodium sulfate, calcium phosphate, or sodium chloride may be used.

As a specific method of Process P1, for example, there may be mentioned a method in which a polymerization initiator is dissolved in a mixed solution (monomer solution) containing a monomer composition and, if necessary, a porogen, and the mixture is suspended in an aqueous medium and heated to a predetermined temperature to carry out polymerization, a method in which a polymerization initiator is dissolved in a mixed solution (monomer solution) containing a monomer composition and, if necessary, a porogen, and the mixture is added to an aqueous medium heated to a predetermined temperature to carry out polymerization, and a method in which a mixed solution (monomer solution) containing a monomer composition and, if necessary, a porogen is suspended in an aqueous medium and heated to a predetermined temperature, and a polymerization initiator is added to carry out polymerization.

As the polymerization initiator, a radical polymerization initiator is preferred. Examples of the radical polymerization initiator include azo-based initiators, peroxide-based initiators, and redox-based initiators, and specifically, examples thereof include azobisisobutyronitrile, azobis methyl isobutyrate, azobis-2,4-dimethylvaleronitrile, benzoyl peroxide, di-tert-butyl peroxide, and benzoyl peroxide-dimethylaniline. The total amount of the polymerization initiator used is usually about 0.01 parts by mass or more and 10 parts by mass or less relative to 100 parts by mass of the total amount of monomers.

The above porogen is used for producing porous particles, is present together with the monomer in polymerization within oil droplets, and has a role of forming pores as a non-polymerizable component. The porogen is not particularly limited as long as it can be easily removed from the porous surface, and examples thereof include various organic solvents and linear polymers soluble in a mixed monomer, and these may be used in combination.

Examples of the above porogen include aliphatic hydrocarbons such as hexane, heptane, octane, nonane, decane, and undecane; alicyclic hydrocarbons such as cyclopentane and cyclohexane; aromatic hydrocarbons such as benzene, toluene, xylene, naphthalene, and ethylbenzene; halogenated hydrocarbons such as carbon tetrachloride, 1,2-dichloroethane, tetrachloroethane, and chlorobenzene; aliphatic alcohols such as butanol, pentanol, hexanol, heptanol, 4-methyl-2-pentanol, and 2-ethyl-1-hexanol; alicyclic alcohols such as cyclohexanol; aromatic alcohols such as 2-phenylethyl alcohol and benzyl alcohol; ketones such as diethyl ketone, methyl isobutyl ketone, diisobutyl ketone, acetophenone, 2-octanone, and cyclohexanone; ethers such as dibutyl ether, diisobutyl ether, anisole, and ethoxybenzene; esters such as isopentyl acetate, butyl acetate, 3-methoxybutyl acetate, and diethyl malonate; and linear polymers such as homopolymers of non-crosslinking vinyl monomers. The porogen may be used alone or in combinations of two or more.

The total amount of the porogen used is usually about 40 parts by mass or more and 600 parts by mass or less relative to 100 parts by mass of the total amount of monomers.

Further, in Process P1, various surfactants including anionic surfactants such as alkyl sulfate salts, alkylaryl sulfate salts, alkyl phosphate salts, and fatty acid salts may be used. Further, polymerization inhibitors such as nitrites (e.g., sodium nitrite), iodide salts (e.g., potassium iodide), tert-butyl pyrocatechol, benzoquinone, picric acid, hydroquinone, copper chloride, and ferric chloride may also be used. Further, a polymerization regulator such as dodecyl mercaptan may be used.

Further, the polymerization temperature in Process P1 may be determined depending on the polymerization initiator, but is usually about 2 to 100°C, and preferably 50 to 100°C. Further, the polymerization time is usually 5 minutes to 48 hours, and preferably 10 minutes to 24 hours.

### -Process P2-

Further, prior to the solid-phase carrier washing process, a process of reacting the porous particles obtained in Process P1 with at least one selected from a crosslinking agent and a hydrophilizing agent (hereinafter also referred to as Process P2) may be carried out. In a case where both the crosslinking agent and the hydrophilizing agent are used, a hydrophilization reaction may be carried out after a crosslinking reaction, or a crosslinking reaction may be carried out after a hydrophilization reaction. Further, the crosslinking reaction and the hydrophilization reaction may be carried out simultaneously.

In a case where a monomer composition containing a functional group-containing monomer is used in Process P1, by the above crosslinking reaction, the crosslinking agent is added to a part of the functional groups contained in the polymer molecule of the porous particles, and a partial structure derived from the crosslinking agent is introduced. As a result, residues of the above functional groups are crosslinked via the partial structure derived from the crosslinking agent.

Further, in a case where a monomer composition containing a functional group-containing monomer is used in Process P1, by the above hydrophilization reaction, the hydrophilizing agent is added to a part of the functional groups contained in the polymer molecule of the porous particles, and a partial structure derived from the hydrophilizing agent is introduced.

The crosslinking agent used in Process P2 is not particularly limited as long as it can introduce a crosslinked structure by reacting with a functional group capable of binding a ligand. A crosslinking agent that can introduce a crosslinked structure by reacting with a functional group capable of binding a ligand and that contains at least two groups represented by -C(=O)-NH- in a molecule thereof is preferred.

In a case where the porous particles obtained in Process P1 have a cyclic ether group, specifically, a crosslinking agent containing at least two groups represented by -C(=O)-NH-NH2 in a molecule thereof as a crosslinkable group, or a crosslinking agent containing at least two groups represented by -C(=O)-NH- in a molecule thereof and also containing at least two carboxyl groups in a molecule thereof as a crosslinkable group can be used.

In a case where the porous particles obtained in Process P1 have a carboxyl group, -C(=O)-O-C(=O)-, a succinimide oxycarbonyl group, a formyl group, or an isocyanate group, specifically, a crosslinking agent containing at least two groups represented by -C(=O)-NH-NH2 in a molecule thereof as a crosslinkable group can be used.

Examples of the crosslinking agent containing at least two groups represented by -C(=O)-NH- in a molecule thereof as described above include dicarboxylic acid dihydrazides such as oxalyl dihydrazide, malonic dihydrazide, succinic dihydrazide, 2,3-dihydroxysuccinic dihydrazide, glutaric dihydrazide, adipic dihydrazide, pimelic dihydrazide, octanedioic dihydrazide, nonanedioic dihydrazide, sebacic dihydrazide, dodecanedioic dihydrazide, phthalic dihydrazide, isophthalic dihydrazide, terephthalic dihydrazide, and quinolinic dihydrazide; tricarboxylic acid trihydrazides such as cyclohexanetricarboxylic acid trihydrazide; and (alkylenebis(imino))bis(oxoalkanoic acids) such as N1,N1-(ethane-1,2-diyl)bis(succinic monoamide). The crosslinking agent may be used alone or in combinations of two or more. Among these crosslinking agents, dicarboxylic acid dihydrazides and (alkylenebis(imino))bis(oxoalkanoic acids) are preferred, and dicarboxylic acid dihydrazides are more preferred, in order to improve liquid permeability, pressure resistance characteristics during liquid passage, and antifouling properties.

Further, in Process P2, a crosslinking agent other than a crosslinking agent containing at least two groups represented by -C(=O)-NH- in a molecule thereof may also be used. Examples of such a crosslinking agent include polyfunctional isocyanate-based crosslinking agents, polyfunctional epoxy-based crosslinking agents, polyfunctional aldehyde-based crosslinking agents, polyfunctional thiol-based crosslinking agents, polyfunctional oxazoline-based crosslinking agents, polyfunctional aziridine-based crosslinking agents, and metal chelate-based crosslinking agents.

The total amount of the crosslinking agent used is preferably 0.01 molar equivalent or more and 0.8 molar equivalent or less, more preferably 0.05 molar equivalent or more and 0.7 molar equivalent or less, and particularly preferably 0.1 molar equivalent or more and 0.6 molar equivalent or less, relative to 1 mol of the functional group derived from the functional group-containing monomer.

As the hydrophilizing agent used in Process P2, in order to improve antifouling properties and low-leakage properties of a proteinaceous ligand, a compound having a total of two or more hydrophilic groups selected from a hydroxyl group and a mercapto group in a molecule thereof is preferred, and a compound having a total of 2 to 4 hydrophilic groups selected from a hydroxyl group and a mercapto group in a molecule thereof is more preferred. Examples thereof include alcohols having a mercapto group in a molecule thereof, such as mercaptoethanol and thioglycerol; and polyhydric alcohols such as glycerol and diglycerol. The hydrophilizing agent may be used alone or in combinations of two or more.

Among these, in order to improve antifouling properties and low-leakage properties of a proteinaceous ligand, alcohols having a mercapto group in a molecule thereof are preferred, and thioglycerol is particularly preferred.

The total amount of the hydrophilizing agent used is preferably 0.5 molar equivalent or more and 10 molar equivalents or less, more preferably 1 molar equivalent or more and 8 molar equivalents or less, and particularly preferably 2 molar equivalents or more and 6 molar equivalents or less, relative to 1 mol of the functional group derived from the functional group-containing monomer.

Process P2 may be carried out in the presence of a basic catalyst. Examples of the basic catalyst include triethylamine, N,N-dimethyl-4-aminopyridine, sodium hydroxide, and diisopropylethylamine, and these may be used alone or in combinations of two or more.

Further, the reaction time in Process P2 is not particularly limited, but is usually about 0.5 to 72 hours, and preferably 0.5 to 48 hours. Further, the reaction temperature may be appropriately selected at or below the boiling point of the solvent, but is usually about 2°C to 100°C.

### -Process P3-

Further, in a case where the ligand is bound to the porous particles via a linker (spacer) in the ligand binding process, a process of reacting the porous particles obtained in Process P1 or Process P2 with a compound that provides a linker (hereinafter also referred to as Process P3) may be carried out prior to the solid-phase carrier washing process.

Examples of the compound that provides a linker include diglycidyl ethers of aliphatic polyhydroxy compounds, such as ethylene glycol diglycidyl ether, diethylene glycol diglycidyl ether, polyethylene glycol diglycidyl ether, 1,2-propylene glycol diglycidyl ether, dipropylene glycol diglycidyl ether, polypropylene glycol diglycidyl ether, 1,4-butanediol diglycidyl ether, neopentyl glycol diglycidyl ether, 1,6-hexanediol diglycidyl ether, and glycerol diglycidyl ether; and polyglycidyl ethers of aliphatic polyhydroxy compounds, such as sorbitol polyglycidyl ether, glycerol polyglycidyl ether, trimethylolpropane polyglycidyl ether, diglycerol polyglycidyl ether, and polyglycerol polyglycidyl ether. Among these, in a case where a hydrophilization reaction is carried out in Process P2, diglycidyl ethers of aliphatic polyhydroxy compounds are preferred.

The linker introduction reaction is preferably carried out in a buffer having a pH of 7 to 14 in order to improve reaction efficiency. Further, the reaction time of the linker introduction reaction is not particularly limited, but is usually about 0.5 to 72 hours. Further, the reaction temperature may be appropriately selected at or below the boiling point of the solvent, but is usually about 2°C to 100°C.

Here, the solid-phase carrier washing process will be described in detail.

The solid-phase carrier washing process is a process of washing with at least one washing liquid selected from the group consisting of a washing liquid containing hydrogen peroxide, a washing liquid containing peracetic acid, and a washing liquid having a pH of 0 or more and 3 or less or a pH of more than 12.5 and 14 or less (excluding a washing liquid containing hydrogen peroxide and a washing liquid containing peracetic acid) (hereinafter also referred to as a "solid-phase carrier washing liquid"). Among the solid-phase carrier washing liquids, in order to increase the hydrophilicity of the surface of the solid-phase carrier and to make a proteinaceous ligand less likely to leak during isolation, a washing liquid having a pH of 0 or more and 3 or less or a pH of more than 12.5 and 14 or less is preferred, and a washing liquid having a pH of more than 12.5 and 14 or less is more preferred.

As the solid-phase carrier washing liquid, an aqueous washing liquid is preferred. The term "aqueous washing liquid" means a washing liquid containing at least water. Examples of the aqueous washing liquid include those containing water or a mixed liquid of water and a lower alcohol. Examples of the lower alcohol include one or more selected from ethanol and isopropanol.

The pH of the washing liquid containing hydrogen peroxide is preferably 4.5 or more and less than 7, more preferably 5 or more and 6.8 or less, particularly preferably 5.5 or more and 6.5 or less, and most preferably 6 or more and 6.5 or less, in order to make a proteinaceous ligand less likely to leak during isolation.

Further, the concentration of hydrogen peroxide in the above washing liquid is preferably 0.001 M or more and 10 M or less, more preferably 0.005 M or more and 5 M or less, and particularly preferably 0.01 M or more and 1 M or less, in order to make a proteinaceous ligand less likely to leak during isolation.

The pH of the washing liquid containing peracetic acid is preferably 1 or more and less than 7, more preferably 2 or more and 6 or less, particularly preferably 3 or more and 5 or less, and most preferably 4 or more and 5 or less, in order to make a proteinaceous ligand less likely to leak during isolation.

Further, the concentration of peracetic acid in the above washing liquid is preferably 0.001 M or more and 5 M or less, more preferably 0.005 M or more and 3 M or less, and particularly preferably 0.01 M or more and 1 M or less, in order to make a proteinaceous ligand less likely to leak during isolation.

In addition to a washing liquid containing hydrogen peroxide and a washing liquid containing peracetic acid, the solid-phase carrier washing liquid includes a washing liquid having a pH of 0 or more and 3 or less and a washing liquid having a pH of more than 12.5 and 14 or less.

The pH of the washing liquid having a pH of 0 or more and 3 or less is preferably 0 or more and 2.5 or less, and more preferably 0 or more and 2 or less, in order to increase the hydrophilicity of the surface of the solid-phase carrier and to make a proteinaceous ligand less likely to leak during isolation.

The pH of the washing liquid having a pH of more than 12.5 and 14 or less is preferably 12.7 or more and 14 or less, more preferably 13 or more and 14 or less, and particularly preferably 13.2 or more and 14 or less, in order to increase the hydrophilicity of the surface of the solid-phase carrier and to make a proteinaceous ligand less likely to leak during isolation.

In a case where the pH of the washing liquid having a pH of more than 12.5 and 14 or less is 13.2 or more and 14 or less, the hydrophilicity of the surface of the solid-phase carrier of the obtained chromatographic carrier is increased, and the proteinaceous ligand becomes particularly less likely to leak during isolation.

As the solid-phase carrier washing liquid, in order to increase the hydrophilicity of the surface of the solid-phase carrier and to make a proteinaceous ligand less likely to leak during isolation, a liquid containing a strongly acidic pH adjusting agent or a strongly basic pH adjusting agent is preferred, and a liquid containing a strongly basic pH adjusting agent is more preferred.

As the strongly acidic pH adjusting agent, an inorganic acid-based strongly acidic pH adjusting agent such as sulfuric acid, hydrochloric acid, or nitric acid is preferred, and hydrochloric acid is more preferred. As the strongly basic pH adjusting agent, an alkali metal hydroxide such as lithium hydroxide, potassium hydroxide, or sodium hydroxide is preferred, and potassium hydroxide and sodium hydroxide are more preferred.

In a case where a strongly basic pH adjusting agent is used, it becomes easy to adjust the pH of the washing liquid to a range of more than 12.5 and 14 or less, and in a case where a strongly acidic pH adjusting agent is used, it becomes easy to adjust the pH of the washing liquid to a range of 0 or more and 5 or less.

Further, the concentration of the strongly acidic pH adjusting agent or the strongly basic pH adjusting agent in the solid-phase carrier washing liquid is preferably 0.001 M or more and 25 M or less, more preferably 0.005 M or more and 10 M or less, further preferably 0.01 M or more and 5 M or less, further preferably 0.05 M or more and 2.5 M or less, further preferably 0.25 M or more and 1.5 M or less, and particularly preferably 0.5 M or more and 1 M or less, in order to make a proteinaceous ligand less likely to leak during isolation.

In a case where the concentration of the strongly acidic pH adjusting agent or the strongly basic pH adjusting agent is 0.25 M or more or 0.5 M or more, particularly excellent low-leakage properties of a proteinaceous ligand are achieved.

The solid-phase carrier washing liquid may contain a buffer or two or more pH adjusting agents in order to adjust to a desired pH or to maintain a target pH.

The washing using the solid-phase carrier washing liquid may be carried out a total of once or a total of two or more times. In order to increase the hydrophilicity of the surface of the solid-phase carrier, to make a proteinaceous ligand less likely to leak during isolation, and to suppress aggregation between carriers, the sum of the number of times is preferably two or more, more preferably 2 to 20 times, further preferably 2 to 15 times, further preferably 2 to 10 times, further preferably 2 to 8 times, and particularly preferably 3 to 4 times. In a case where washing is carried out a total of two or more times, the type of the solid-phase carrier washing liquid used each time may be the same or different, but is preferably the same.

In a case where the number of times of washing using the solid-phase carrier washing liquid is 3 or more in total, particularly excellent low-leakage properties of a proteinaceous ligand are achieved. Further, in a case where the sum of the number of times of washing using the solid-phase carrier washing liquid is 4 or less, particularly excellent low aggregation properties are achieved.

The amount of the solid-phase carrier washing liquid used per one washing is preferably 40 parts by volume or more, more preferably 60 parts by volume or more, and particularly preferably 80 parts by volume or more, and is also preferably 1000 parts by volume or less, more preferably 500 parts by volume or less, and particularly preferably 300 parts by volume or less, relative to 100 parts by volume of the dry solid content of the solid-phase carrier. Specifically, the range is preferably 40 parts by volume or more and 1000 parts by volume or less, more preferably 60 parts by volume or more and 500 parts by volume or less, and particularly preferably 80 parts by volume or more and 300 parts by volume or less, relative to 100 parts by volume of the dry solid content of the solid-phase carrier.

The washing temperature is not particularly limited, but is usually in a range of 10°C to 50°C, and preferably in a range of 15°C to 45°C.

Further, either batch washing or continuous washing may be used.

The washing method using the solid-phase carrier washing liquid is not particularly limited. Examples thereof include liquid-passing washing, stirring washing, and standing washing. Among these, in order to increase the hydrophilicity of the surface of the solid-phase carrier, to make a proteinaceous ligand less likely to leak during isolation, to increase dynamic binding capacity, and to suppress aggregation between carriers, liquid-passing washing and stirring washing are preferred, in order to further improve replacement efficiency, liquid-passing washing is more preferred, and in order to suppress aggregation between carriers, stirring washing is more preferred, and a combination of liquid-passing washing and stirring washing is particularly preferred. In a case where liquid-passing washing and stirring washing are carried out, the order and number of times thereof are not particularly limited. It is preferred that stirring washing is carried out after liquid-passing washing and that each is carried out at least once. In a case where liquid-passing washing and stirring washing are carried out in combination as the solid-phase carrier washing process, particularly excellent low-leakage properties of a proteinaceous ligand, low aggregation properties, and replacement efficiency are achieved.

In a case where washing is carried out a total of two or more times, the type of washing method used each time may be the same or different.

The liquid-passing washing is not particularly limited as long as washing in which the solid-phase carrier washing liquid is passed through a solid-phase carrier bed one or more times. In a case where liquid-passing washing is carried out, as the solid-phase carrier washing process, a process including the following solid-phase carrier bed formation process and solid-phase carrier liquid-passing washing process is preferred.
(Solid-phase carrier bed formation process) a process of filling a solid-phase carrier into a container to form a solid-phase carrier bed
(Solid-phase carrier liquid-passing washing process) a process of washing the solid-phase carrier bed formed in the solid-phase carrier bed formation process by passing at least one washing liquid selected from the group consisting of a washing liquid containing hydrogen peroxide, a washing liquid containing peracetic acid, and a washing liquid having a pH of 0 or more to 3 or less or a pH of more than 12.5 and 14 or less (excluding a washing liquid containing hydrogen peroxide and a washing liquid containing peracetic acid) therethrough one or more times

### (Solid-phase Carrier Bed Formation Process)

In the present invention, the "solid-phase carrier bed" means a solid-phase carrier solid layer, and may be either dry or wet. As a specific method for forming the solid-phase carrier bed, for example, there may be mentioned a method in which a slurry of the solid-phase carrier (for example, one using, as a dispersion medium, water, an alcohol (such as ethanol or isopropanol), a mixed liquid of water and an alcohol, or a buffer (such as a carbonate buffer)) is introduced into a container, the solid content of the solid-phase carrier is allowed to settle at the bottom of the container by gravity sedimentation or centrifugal sedimentation, and the liquid phase of the slurry is removed by a solid-liquid separation operation (for example, decantation, centrifugation, or filtration).

Further, as the container, a solid-liquid separator is preferred because the washing liquid can be directly passed through the solid-phase carrier bed formed in the solid-phase carrier bed formation process in the subsequent solid-phase carrier liquid-passing washing process, which is simple. Specifically, examples thereof include a column container with a filter, a funnel with a filter, a centrifuge with a filter, and a filter reactor.

The content ratio of the solid-phase carrier in the slurry is preferably 5 vol% or more, more preferably 15 vol% or more, and particularly preferably 30 vol% or more, and is also preferably 80 vol% or less, more preferably 75 vol% or less, and particularly preferably 70 vol% or less. Specifically, the range is preferably 5 vol% or more and 80 vol% or less, more preferably 15 vol% or more and 75 vol% or less, and particularly preferably 30 vol% or more and 70 vol% or less in the slurry.

The volume percentage of the carrier in the slurry can be calculated, for example, by filling 200 mL of the slurry into a 250 mL glass graduated cylinder manufactured by Corning (conforming to JIS R3505 Class A), allowing it to stand, and dividing the sedimentation volume after 3 hours by the volume of the slurry filled in the graduated cylinder.

### (Solid-phase Carrier Liquid-passing Washing Process)

In the present invention, "liquid-passing washing" of the solid-phase carrier means passing a washing liquid through a solid-phase carrier bed, and, for example, washing is carried out by allowing the washing liquid to pass from one direction of the solid-phase carrier bed to the opposite direction. Specifically, washing can be carried out using a solid-liquid separator such as a column container with a filter, a filter plate, a funnel with a filter, a Buchner funnel, a Nutsch filter, a centrifuge with a filter, or a filter reactor. The washing liquid flowed into the solid-phase carrier bed may be incubated for preferably 30 seconds to 3 hours, more preferably 2 minutes to 60 minutes, and then flowed out.

The number of times of liquid-passing washing in the solid-phase carrier liquid-passing washing process is preferably 1 or more, more preferably 1 to 20 times, further preferably 1 to 10 times, further preferably 1 to 5 times, further preferably 2 to 5 times, and particularly preferably 2 to 3 times, in order to increase the hydrophilicity of the surface of the solid-phase carrier, to make a proteinaceous ligand less likely to leak during isolation, and to suppress aggregation between carriers.

In a case where the number of times of liquid-passing washing is two or more, particularly excellent low-leakage properties of a proteinaceous ligand are achieved. In a case where the number of times of liquid-passing washing is 3 or less, particularly excellent low aggregation properties are achieved.

The type of the solid-phase carrier washing liquid and the amount of the solid-phase carrier washing liquid used per one washing are as described above.

The liquid-passing washing may be washing using gravitational flow, washing by pressurized flow, or washing by reduced pressure.

### (Solid-phase Carrier Stirring Washing Process)

The stirring washing is not particularly limited as long as washing in which the solid-phase carrier is stirred in the solid-phase carrier washing liquid. In order to increase the hydrophilicity of the surface of the solid-phase carrier, to make a proteinaceous ligand less likely to leak during isolation, to increase dynamic binding capacity, and to suppress aggregation between carriers, it is preferred that the solid-phase carrier after the solid-phase carrier liquid-passing washing process is subjected to stirring washing one or more times in the solid-phase carrier washing liquid.

The number of times of stirring washing in the solid-phase carrier stirring washing process is preferably 1 or more, more preferably 1 to 20 times, further preferably 1 to 10 times, further preferably 1 to 5 times, further preferably one or two times, and particularly preferably 1 time, in order to increase the hydrophilicity of the surface of the solid-phase carrier, to make a proteinaceous ligand less likely to leak during isolation, and to suppress aggregation between carriers.

In a case where the number of times of stirring washing is 1 or more, particularly excellent low aggregation properties are achieved. In addition, in a case where the number of times of stirring washing is two or less, particularly excellent low-leakage properties of a proteinaceous ligand are achieved.

The stirring speed in the solid-phase carrier stirring washing process is preferably 10 to 150 rpm, more preferably 20 to 100 rpm, and particularly preferably 30 to 80 rpm.

The stirring time per one stirring washing in the solid-phase carrier stirring washing process is preferably 30 seconds to 300 minutes, more preferably 1 minute to 180 minutes, further preferably 2 minutes to 120 minutes, and particularly preferably 5 minutes to 90 minutes.

The type of the solid-phase carrier washing liquid and the amount of the solid-phase carrier washing liquid used per one washing are as described above.

In a case where the solid-phase carrier liquid-passing washing process is carried out using a solid-liquid separator, continuous stirring washing can be carried out by performing the solid-phase carrier stirring washing process with a filtrate discharge outlet of the solid-liquid separator used in the solid-phase carrier liquid-passing washing process kept open, and batch stirring washing can be carried out by performing the solid-phase carrier stirring washing process using the solid-liquid separator with the filtrate discharge outlet closed as a container.

After completion of the solid-phase carrier washing, prior to the ligand binding process, the washing liquid may be removed or the solid-phase carrier may be dispersed in water or a mixed liquid of water and a lower alcohol, if necessary.

### (Ligand Binding Process)

A ligand binding process is a process of binding a proteinaceous ligand to the solid-phase carrier washed in the solid-phase carrier washing process

As the proteinaceous ligand, one or more ligands selected from protein A, protein G, protein L, and derivatives thereof are preferred, and in order to increase dynamic binding capacity and to make a proteinaceous ligand less likely to leak during isolation, the protein A and modified protein A are preferred, and the modified protein A is more preferred.

The protein A includes five domains, E, D, A, B, and C, having binding ability to immunoglobulin; among the above ligands, a derivative of the protein A having modified domains of the B and C domains is preferred, and a derivative of the protein A having a modified domain of the C domain is more preferred.

Further, among the proteinaceous ligands, in order to increase dynamic binding capacity and to make a proteinaceous ligand less likely to leak during isolation, a proteinaceous ligand having an amino acid sequence in which at least one or two or more substitutions selected from the following (a) to (i) are introduced into an amino acid sequence having 85% or more sequence identity to the amino acid sequence represented by SEQ ID NO. 1 (C domain of the protein A) is preferred. Among such proteinaceous ligands, those having an amino acid sequence in which two or more substitutions selected from the following (a) to (i) are introduced are preferred, those having an amino acid sequence in which 2 to 9 substitutions selected from the following (a) to (i) are introduced are more preferred, and those having an amino acid sequence in which 2 to 6 substitutions selected from the following (a) to (i) are introduced are particularly preferred. Further, a ligand having two or more such amino acid sequences is preferred, a ligand having 2 to 12 such amino acid sequences is more preferred, and a ligand having 4 to 7 such amino acid sequences is particularly preferred. When two or more amino acid sequences are contained, the amino acid sequences may be homologous or heterologous.

(a) Substitution of the amino acid residue at a position corresponding to position 1 of the amino acid sequence of SEQ ID NO. 1 with a valine residue
(b) Substitution of the amino acid residue at a position corresponding to position 3 of the amino acid sequence of SEQ ID NO. 1 with an alanine residue
(c) Substitution of the amino acid residue at a position corresponding to position 6 of the amino acid sequence of SEQ ID NO. 1 with an alanine residue or an aspartic acid residue
(d) Substitution of the amino acid residue at a position corresponding to position 9 of the amino acid sequence of SEQ ID NO. 1 with an alanine residue
(e) Substitution of the amino acid residue at a position corresponding to position 11 of the amino acid sequence of SEQ ID NO. 1 with an alanine residue, a glutamine residue, or a glutamic acid residue
(f) Substitution of the amino acid residue at a position corresponding to position 23 of the amino acid sequence of SEQ ID NO. 1 with a leucine residue
(g) Substitution of the amino acid residue at a position corresponding to position 29 of the amino acid sequence of SEQ ID NO. 1 with an alanine residue
(h) Substitution of the amino acid residue at a position corresponding to position 43 of the amino acid sequence of SEQ ID NO. 1 with an alanine residue
(i) Substitution of the amino acid residue at a position corresponding to position 49 of the amino acid sequence of SEQ ID NO. 1 with an arginine residue

Examples of the means for substitution of an amino acid residue include known means such as site-specific mutation of a polynucleotide encoding a domain.

Here, "85% or more sequence identity" with respect to an amino acid sequence preferably means 90% or more sequence identity, more preferably 95% or more sequence identity, further preferably 97% or more sequence identity, further preferably 98% or more sequence identity, and particularly preferably 99% or more sequence identity.

In the present specification, a "corresponding position" on an amino acid sequence can be determined by aligning a target sequence and a reference sequence (for example, the amino acid sequence of SEQ ID NO. 1) to give the highest sequence identity to conserved amino acid residues present in each amino acid sequence. The alignment can be carried out using a known algorithm, and the procedure is well known to those skilled in the art. For example, the alignment can be carried out using the Clustal W multiple alignment program (Thompson, J. D. et al., 1994, Nucleic Acids Res., 22:4673-4680) with default settings. Clustal W can be used, for example, on the websites of the European Bioinformatics Institute (EBI [www.ebi.ac.uk/index.html]) or the DNA Data Bank of Japan (DDBJ [www.ddbj.nig.ac.jp/index.html]) operated by the National Institute of Genetics.

In the present specification, amino acid residues are also represented by the following abbreviations: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), valine (Val or V), and any amino acid residue (Xaa or X). Further, in the present specification, the amino acid sequence of a peptide is described, according to a conventional method, such that the amino terminus (hereinafter referred to as the N-terminus) is positioned on the left side and the carboxyl terminus (hereinafter referred to as the C-terminus) is positioned on the right side.

In the present specification, the positions "before" and "after" a specific position in an amino acid sequence mean positions adjacent to the N-terminus side and the C-terminus side of the specific position, respectively. For example, in a case where an amino acid residue is inserted at positions "before" and "after" a specific position, the inserted amino acid residue is arranged at positions adjacent to the N-terminus side and the C-terminus side of the specific position.

In a preferred embodiment, the proteinaceous ligand is produced by introducing one or two or more substitutions selected from the above (a) to (i) into a proteinaceous ligand (parent domain) having an amino acid sequence with 85% or more sequence identity to the amino acid sequence represented by SEQ ID NO. 1.

Examples of the means for substituting a proteinaceous ligand (parent domain) having an amino acid sequence with 85% or more sequence identity to the amino acid sequence represented by SEQ ID NO. 1 include a method of introducing a mutation into a polynucleotide encoding the parent domain so that substitution of a desired amino acid residue occurs. Specific methods for introducing a mutation into a polynucleotide include site-specific mutation, homologous recombination, and splicing by overlap extension (SOE)-PCR (Gene, 1989, 77:61-68), and detailed procedures thereof are well known to those skilled in the art.

The produced ligand has immunoglobulin-binding activity and functions as an immunoglobulin-binding domain.

Preferred examples of the proteinaceous ligand include those in which two or more amino acid sequences, each having at least one or two or more substitutions selected from the following (a) to (i) in an amino acid sequence having 85% or more sequence identity to the amino acid sequence represented by SEQ ID NO. 1 (C domain of protein A), are linearly linked. The term "linearly linked" with respect to amino acid sequences means a structure in which two or more amino acid sequences are serially linked with or without a linker. For example, in a case where a linker is present, "linearly linked" means a structure in which the C-terminus of one amino acid sequence and the N-terminus of another amino acid sequence are serially linked via a linker, whereas in a case where a linker is not present, "linearly linked" means a structure in which the C-terminus of one amino acid sequence and the N-terminus of another amino acid sequence are serially linked by a peptide bond.

Specifically, examples thereof include a modified protein A that is a trimer to pentamer of amino acid sequence domains represented by SEQ ID NOs. 2 to 4 and amino acid sequence domains having 85% or more sequence identity thereto (provided that they have 85% or more sequence identity to the amino acid sequence represented by SEQ ID NO. 1). In order to make a proteinaceous ligand less likely to leak during isolation, a modified protein A that is a trimer to pentamer of amino acid sequence domains represented by SEQ ID NOs. 2 to 3 and amino acid sequence domains having 85% or more sequence identity thereto (provided that they have 85% or more sequence identity to the amino acid sequence represented by SEQ ID NO. 1) is preferred.

The binding amount of the proteinaceous ligand is preferably 10 mg or more and 300 mg or less, and more preferably 25 mg or more and 150 mg or less, per 1 g of the dry weight of the solid-phase carrier, in order to increase dynamic binding capacity.

The binding of the proteinaceous ligand to the solid-phase carrier in the ligand binding process may be carried out in the same manner as a conventional method. As the ligand binding method, a chemical binding method is preferred. For example, a method of binding a ligand to a functional group capable of binding the ligand may be mentioned. This method may be carried out with reference to the descriptions of, for example, WO 2015/119255 and WO 2015/041218. Specifically, a method of binding a cyclic ether group, a carboxyl group, -C(=O)-O-C(=O)-, a formyl group, or the like of the solid-phase carrier to an amino group or the like of the ligand may be mentioned. The ligand binding reaction is preferably carried out in a buffer having a pH of 7 to 14 in order to improve reaction efficiency. Further, the reaction time of the ligand binding reaction is not particularly limited, but is usually about 0.1 to 72 hours. Further, the reaction temperature may be appropriately selected at or below the boiling point of the solvent, but is usually about 2°C to 100°C.

Further, binding may be carried out using a method for controlling the orientation of a ligand (U.S. Patent No. 6,399,750; Ljungquist C. et al., Eur. J. Biochem., 1989, Vol. 186, pp. 557-561), a method of binding a ligand to a solid-phase carrier via a linker (spacer) (U.S. Patent No. 5,260,373; JP 2010-133733 A; JP 2010-133734 A), a method of accumulating a ligand on a solid-phase carrier by an associative group (JP 2011-256176 A), or the like.

As the production method for a chromatographic carrier of the present invention, in order to improve antifouling properties and to make a proteinaceous ligand less likely to leak during isolation, a method is preferred in which a ligand-bound carrier hydrophilization process is further provided between the ligand binding process and the ligand-bound carrier washing process, and the hydrophilic group-containing ligand-bound carrier obtained in the ligand-bound carrier hydrophilization process is used as the ligand-bound carrier after the ligand binding process in the ligand-bound carrier washing process.

(Ligand-bound carrier hydrophilization process) a process of reacting a carrier to which a ligand has been bound in the ligand binding process with a compound having a total of two or more hydrophilic groups selected from a hydroxyl group and a mercapto group in a molecule thereof

As the compound having a total of two or more hydrophilic groups in a molecule thereof used in the ligand-bound carrier hydrophilization process, in order to improve antifouling properties and to make a proteinaceous ligand less likely to leak during isolation, a compound having a total of 2 to 4 hydrophilic groups selected from a hydroxyl group and a mercapto group in a molecule thereof is preferred. Examples thereof include alcohols having a mercapto group in a molecule thereof, such as mercaptoethanol and thioglycerol; and polyhydric alcohols such as glycerol and diglycerol. The compound having a total of two or more hydrophilic groups in a molecule thereof may be used alone or in combinations of two or more.

Among these, in order to improve antifouling properties and to make a proteinaceous ligand less likely to leak during isolation, alcohols having a mercapto group in a molecule thereof are preferred, and thioglycerol is particularly preferred.

The total amount of the compound having a total of two or more hydrophilic groups in a molecule thereof used in the ligand-bound carrier hydrophilization process is preferably 1 part by mass or more and 1000 parts by mass or less, more preferably 10 parts by mass or more and 800 parts by mass or less, and particularly preferably 100 parts by mass or more and 600 parts by mass or less, relative to 100 parts by mass of the ligand-bound carrier (dry solid content).

The ligand-bound carrier hydrophilization process may be carried out in the presence of a basic catalyst. Examples of the basic catalyst include triethylamine, N,N-dimethyl-4-aminopyridine, sodium hydroxide, and diisopropylethylamine, and these may be used alone or in combinations of two or more.

Further, the reaction time of the ligand-bound carrier hydrophilization process is not particularly limited, but is usually about 0.5 to 72 hours, and preferably 0.5 to 48 hours. Further, the reaction temperature may be appropriately selected at or below the boiling point of the solvent, but is usually about 2°C to 100°C.

### (Ligand-bound Carrier Washing Process)

The ligand-bound carrier washing process is a process of washing the carrier obtained in the ligand binding process.

The ligand-bound carrier washing process is preferably carried out by washing with a washing liquid (hereinafter also referred to as a "ligand-bound carrier washing liquid").

As the ligand-bound carrier washing liquid, an aqueous washing liquid is preferred. The term "aqueous washing liquid" means a washing liquid containing at least water. Examples of the aqueous washing liquid include those containing water or a mixed liquid of water and a lower alcohol. Examples of the lower alcohol include one or more selected from ethanol and isopropanol.

The pH of the ligand-bound carrier washing liquid is, in order to increase the hydrophilicity of the surface of the solid-phase carrier, to make a proteinaceous ligand less likely to leak during isolation, and to increase dynamic binding capacity, preferably more than 3, more preferably 6 or more, further preferably 7 or more, further preferably 8.5 or more, further preferably 9.5 or more, and particularly preferably 10.5 or more. In addition, the pH is preferably 12.5 or less, more preferably 12 or less, and particularly preferably 11.8 or less, in order to increase dynamic binding capacity. Specifically, the range is preferably more than pH 3 and 12.5 or less, more preferably 6 or more and 12.5 or less, further preferably 7 or more and 12.5 or less, further preferably 8.5 or more and 12 or less, further preferably 9.5 or more and 12 or less, further preferably 10.5 or more and 12 or less, and particularly preferably 10.5 or more and 11.8 or less.

According to the production method for a chromatographic carrier of the present invention, even in a case where the pH of the washing liquid used in the ligand-bound carrier washing process is in the above mild pH range, a chromatographic carrier in which a proteinaceous ligand is less likely to leak during isolation can be obtained. Further, in a case where the pH of the washing liquid used in the ligand-bound carrier washing process is set in the above mild pH range, dynamic binding capacity is increased, and thus both excellent dynamic binding capacity and excellent low-leakage properties are achieved.

In a case where the pH of the ligand-bound carrier washing liquid is 8.5 or more, 9.5 or more, or 10.5 or more, particularly excellent dynamic binding capacity and low-leakage properties of a proteinaceous ligand are achieved. Further, in a case where the pH of the ligand-bound carrier washing liquid is 12 or less or 11.8 or less, particularly excellent dynamic binding capacity is achieved.

The ligand-bound carrier washing liquid preferably contains a pH adjusting agent in order to be in the above pH range.

The pH adjusting agent is broadly classified into an acidic pH adjusting agent and a basic pH adjusting agent. Specifically, examples thereof include inorganic acid-based strongly acidic pH adjusting agents such as sulfuric acid, hydrochloric acid, and nitric acid; inorganic acid-based weakly acidic pH adjusting agents such as sodium dihydrogen phosphate, carbonic acid, phosphoric acid, hydrogen fluoride, and hydrogen sulfide; organic acid-based weakly acidic pH adjusting agents such as acetic acid and oxalic acid; inorganic base-based strongly basic pH adjusting agents such as alkali metal hydroxides, sodium carbonate, and trisodium phosphate; organic base-based strongly basic pH adjusting agents such as triethylamine; inorganic base-based weakly basic pH adjusting agents such as sodium hydrogen carbonate, disodium hydrogen phosphate, ammonia, copper hydroxide, magnesium hydroxide, zinc hydroxide, iron hydroxide, and aluminum hydroxide; and organic base-based weakly basic pH adjusting agents such as diethanolamine.

Examples of the alkali metal hydroxide include lithium hydroxide, potassium hydroxide, and sodium hydroxide.

The concentration of the pH adjusting agent in the ligand-bound carrier washing liquid is preferably 0.001 M or more and 10 M or less, more preferably 0.005 M or more and 2.5 M or less, further preferably 0.01 M or more and 0.5 M or less, and particularly preferably 0.05 M or more and 0.3 M or less, in order to make a proteinaceous ligand less likely to leak during isolation.

In a case where the concentration of the pH adjusting agent is 0.05 M or more, particularly excellent low-leakage properties of a proteinaceous ligand are achieved. In a case where the concentration of the pH adjusting agent is 0.3 M or less, particularly excellent dynamic binding capacity is achieved.

The ligand-bound carrier washing liquid may contain a buffer or two or more pH adjusting agents in order to adjust to a desired pH or to maintain a target pH.

The washing using the ligand-bound carrier washing liquid may be carried out a total of once or a total of two or more times. In order to increase the hydrophilicity of the surface of the solid-phase carrier, to make a proteinaceous ligand less likely to leak during isolation, to further improve replacement efficiency, and to suppress aggregation between carriers, the sum of the number of times is preferably two or more, more preferably 2 to 20 times, further preferably 2 to 15 times, further preferably 2 to 10 times, further preferably 2 to 8 times, and particularly preferably 3 to 4 times. In a case where washing is carried out a total of two or more times, the type of the ligand-bound carrier washing liquid used each time may be the same or different, but is preferably the same.

In a case where the number of times of washing using the ligand-bound carrier washing liquid is 3 or more in total, replacement efficiency is improved, and the obtained chromatographic carrier exhibits particularly excellent low-leakage properties of a proteinaceous ligand. In a case where the number of times of washing using the ligand-bound carrier washing liquid is 4 or less in total, particularly excellent low aggregation properties are achieved.

The amount of the ligand-bound carrier washing liquid used per one washing is preferably 40 parts by volume or more, more preferably 60 parts by volume or more, and particularly preferably 80 parts by volume or more, and is also preferably 1000 parts by volume or less, more preferably 500 parts by volume or less, and particularly preferably 300 parts by volume or less, relative to 100 parts by volume of the dry solid content of the ligand-bound carrier. Specifically, the range is preferably 40 parts by volume or more and 1000 parts by volume or less, more preferably 60 parts by volume or more and 500 parts by volume or less, and particularly preferably 80 parts by volume or more and 300 parts by volume or less, relative to 100 parts by volume of the dry solid content of the ligand-bound carrier.

The washing temperature is not particularly limited, but is usually in a range of 10°C to 50°C, and preferably in a range of 15°C to 45°C.

Further, either batch washing or continuous washing may be used.

The method of washing using the ligand-bound carrier washing liquid is not particularly limited. Examples thereof include liquid-passing washing, stirring washing, and standing washing. Among these, in order to increase the hydrophilicity of the surface of the solid-phase carrier, to make a proteinaceous ligand less likely to leak during isolation, to increase dynamic binding capacity, and to suppress aggregation between carriers, liquid-passing washing and stirring washing are preferred, in order to further improve replacement efficiency, liquid-passing washing is more preferred, and in order to suppress aggregation between carriers, stirring washing is more preferred, and a combination of liquid-passing washing and stirring washing is particularly preferred. In a case where liquid-passing washing and stirring washing are carried out, the order and number of times thereof are not particularly limited. It is preferred that stirring washing is carried out after liquid-passing washing and that each is carried out at least once.

In a case where liquid-passing washing and stirring washing are carried out in combination as the ligand-bound carrier washing process, particularly excellent low-leakage properties of a proteinaceous ligand, low aggregation properties, and replacement efficiency are achieved.

In a case where washing is carried out a total of two or more times, the type of washing method used each time may be the same or different.

The liquid-passing washing is not particularly limited as long as washing in which the ligand-bound carrier washing liquid is passed through a ligand-bound carrier bed one or more times. In a case where liquid-passing washing is carried out, as the ligand-bound carrier washing process, a process including the following ligand-bound carrier bed formation process and ligand-bound carrier liquid-passing washing process is preferred.

(Ligand-bound carrier bed formation process) a process of filling a ligand-bound carrier into a container to form a ligand-bound carrier bed

(Ligand-bound carrier liquid-passing washing process) a process of subjecting the ligand-bound carrier bed formed in the ligand-bound carrier bed formation process to liquid-passing washing one or more times with a ligand-bound carrier washing liquid (preferably a washing liquid having a pH of more than 3 and 12.5 or less)

### (Ligand-bound Carrier Bed Formation Process)

In the present invention, the "ligand-bound carrier bed" means a solid layer of the ligand-bound carrier, and may be either dry or wet. As a specific method for forming the ligand-bound carrier bed, for example, there may be mentioned a method in which a slurry of the ligand-bound carrier (for example, one using, as a dispersion medium, water, an alcohol (such as ethanol or isopropanol), a mixed liquid of water and an alcohol, or a buffer (such as a carbonate buffer)) is introduced into a container, the solid content of the ligand-bound carrier is allowed to settle at the bottom of the container by gravity sedimentation or centrifugal sedimentation, and the liquid phase of the slurry is removed by a solid-liquid separation operation (for example, decantation, centrifugation, or filtration).

Further, as the container, a solid-liquid separator is preferred because the washing liquid can be directly passed through the ligand-bound carrier bed formed in the ligand-bound carrier bed formation process in the subsequent ligand-bound carrier liquid-passing washing process, thereby being simple. Specifically, examples thereof include a column container with a filter, a funnel with a filter, a centrifuge with a filter, and a filter reactor.

The content ratio of the ligand-bound carrier in the slurry is preferably 5 vol% or more, more preferably 15 vol% or more, and particularly preferably 30 vol% or more, and is also preferably 80 vol% or less, more preferably 75 vol% or less, and particularly preferably 70 vol% or less. Specifically, the range is preferably 5 vol% or more and 80 vol% or less, more preferably 15 vol% or more and 75 vol% or less, and particularly preferably 30 vol% or more and 70 vol% or less in the slurry.

The volume percentage of the carrier in the slurry can be calculated, for example, by filling 200 mL of the slurry into a 250 mL glass graduated cylinder manufactured by Corning (conforming to JIS R3505 Class A), allowing it to stand, and dividing the sedimentation volume after 3 hours by the volume of the slurry filled in the graduated cylinder.

### (Ligand-bound Carrier Liquid-passing Washing Process)

In the present invention, "liquid-passing washing" of the ligand-bound carrier means passing a washing liquid through a ligand-bound carrier bed, and, for example, washing is carried out by allowing the washing liquid to pass from one direction of the ligand-bound carrier bed to the opposite direction. Specifically, washing can be carried out using a solid-liquid separator such as a column container with a filter, a filter plate, a funnel with a filter, a Buchner funnel, a Nutsch filter, a centrifuge with a filter, or a filter reactor. The washing liquid introduced into the ligand-bound carrier bed may be incubated, preferably for 30 seconds to 3 hours, more preferably for 2 minutes to 60 minutes, and then discharged.

The number of times of liquid-passing washing in the ligand-bound carrier liquid-passing washing process is preferably 1 or more, more preferably 1 to 20 times, further preferably 1 to 10 times, further preferably 1 to 5 times, further preferably 2 to 5 times, and particularly preferably 2 to 3 times, in order to increase the hydrophilicity of the surface of the solid-phase carrier, to make a proteinaceous ligand less likely to leak during isolation, to further improve replacement efficiency, and to suppress aggregation between carriers.

In a case where the number of times of liquid-passing washing is 2 or more, replacement efficiency is improved, and the obtained chromatographic carrier exhibits particularly excellent low-leakage properties of a proteinaceous ligand. In a case where the number of times of liquid-passing washing is 3 or less, particularly excellent low aggregation properties are achieved.

The sum of the number of times of liquid-passing washing in the solid-phase carrier liquid-passing washing process and the ligand-bound carrier liquid-passing washing process is preferably 2 or more, more preferably 2 to 30 times, further preferably 2 to 16 times, further preferably 2 to 8 times, further preferably 3 to 6 times, and particularly preferably 4 to 5 times, in order to increase the hydrophilicity of the surface of the solid-phase carrier, to make a proteinaceous ligand less likely to leak during isolation, to further improve replacement efficiency, and to suppress aggregation between carriers.

In a case where the sum of the number of times of liquid-passing washing in the solid-phase carrier liquid-passing washing process and the ligand-bound carrier liquid-passing washing process is 4 or more, replacement efficiency is improved, and the obtained chromatographic carrier exhibits particularly excellent low-leakage properties of a proteinaceous ligand. In a case where the sum of the number of times of liquid-passing washing in the solid-phase carrier liquid-passing washing process and the ligand-bound carrier liquid-passing washing process is 5 or less, particularly excellent low aggregation properties are achieved.

The type of the ligand-bound carrier washing liquid and the amount of the ligand-bound carrier washing liquid used per one washing are as described above.

The liquid-passing washing may be washing using gravitational flow, washing by pressurized flow, or washing by reduced pressure.

### (Ligand-bound Carrier Stirring Washing Process)

The stirring washing is not particularly limited as long as washing in which the ligand-bound carrier is stirred in the ligand-bound carrier washing liquid. In order to enhance hydrophilicity of a surface of the solid-phase carrier, to make it difficult for a proteinaceous ligand to leak out during isolation, to increase a dynamic binding capacity, and to suppress aggregation between carriers, it is preferable that the ligand-bound carrier after the ligand-bound carrier liquid-passing washing process be subjected to stirring washing one or more times in a ligand-bound carrier washing liquid (preferably a washing liquid having a pH of more than 3 and 12.5 or less).

The number of times of stirring washing in the ligand-bound carrier stirring washing process is preferably 1 or more, more preferably 1 to 20 times, further preferably 1 to 10 times, further preferably 1 to 5 times, further preferably one or two times, and particularly preferably 1 time, in order to increase the hydrophilicity of the surface of the solid-phase carrier, to make a proteinaceous ligand less likely to leak during isolation, and to suppress aggregation between carriers.

In a case where the number of times of stirring washing is 1 or more, particularly excellent low aggregation properties are achieved. In a case where the number of times of stirring washing is two or less, particularly excellent low-leakage properties of a proteinaceous ligand are achieved.

The sum of the number of times of stirring washing in the solid-phase carrier stirring washing process and the ligand-bound carrier stirring washing process is preferably 2 or more, more preferably 2 to 30 times, further preferably 2 to 16 times, further preferably 2 to 8 times, further preferably 2 to 6 times, and particularly preferably 2 to 3 times, in order to increase the hydrophilicity of the surface of the solid-phase carrier, to make a proteinaceous ligand less likely to leak during isolation, to further improve replacement efficiency, and to suppress aggregation between carriers.

The stirring speed in the ligand-bound carrier stirring washing process is preferably 10 to 150 rpm, more preferably 20 to 100 rpm, and particularly preferably 30 to 80 rpm.

The stirring time per one stirring washing in the ligand-bound carrier stirring washing process is preferably 30 seconds to 300 minutes, more preferably 1 minute to 180 minutes, further preferably 2 minutes to 60 minutes, and particularly preferably 5 minutes to 30 minutes.

The type of the ligand-bound carrier washing liquid and the amount of the ligand-bound carrier washing liquid used per one washing are as described above.

In a case where the ligand-bound carrier liquid-passing washing process is carried out using a solid-liquid separator, continuous stirring washing can be carried out by performing the ligand-bound carrier stirring washing process with a filtrate discharge outlet of the solid-liquid separator used in the ligand-bound carrier liquid-passing washing process kept open, and batch stirring washing can be carried out by performing the ligand-bound carrier stirring washing process using the solid-liquid separator with the filtrate discharge outlet closed as a container.

The reaction product obtained in each of the above processes may be purified by separation means such as filtration and washing. Further, classification may be carried out.

According to the production method for a chromatographic carrier of the present invention, a chromatographic carrier having a large dynamic binding capacity for an antibody or a fragment thereof, in which a proteinaceous ligand is less likely to leak during isolation, and in which aggregation between carriers is less likely to occur can be produced. Further, replacement efficiency is excellent, and production is straightforward. Notably, although the production method for a chromatographic carrier of the present invention involves washing a solid-phase carrier prior to binding of a ligand, it can unexpectedly produce a chromatographic carrier having a large dynamic binding capacity for an antibody or a fragment thereof.

Further, it is possible to use washing liquids having various pH values as the washing liquid used in the ligand-bound carrier washing process. For example, even in a case where a washing liquid having a pH of more than 3 and 12.5 or less is used in the ligand-bound carrier washing process, a chromatographic carrier in which a proteinaceous ligand is less likely to leak during isolation is obtained.

In the present specification, the term "antibody" is a concept that includes, for example, immunoglobulins of any class such as IgG, IgA, IgD, IgE, and IgM, and subclasses thereof, as well as variants thereof. Further, in the present specification, the term "antibody" may also refer to a chimeric antibody such as a humanized antibody, an antibody complex, or other immunoglobulin-modified forms including an antigen recognition site.

Further, in the present specification, the term "fragment of an antibody" may be either a fragment of an antibody including an antigen recognition site or a fragment of an antibody not including an antigen recognition site. Examples of fragments of an antibody not including an antigen recognition site include proteins consisting only of the Fc region of an immunoglobulin, Fc fusion proteins, and variants or modified forms thereof.

The volume average particle diameter of the chromatographic carrier obtained as described above is preferably 40 to 150 µm, and more preferably 50 to 100 µm. The coefficient of variation of the volume average particle diameter is preferably 40% or less, and more preferably 30% or less.

The specific surface area of the chromatographic carrier is preferably 1 to 500 m²/g, and more preferably 10 to 300 m²/g.

The volume average pore diameter of the chromatographic carrier is preferably 10 to 300 nm.

The above volume average particle diameter, coefficient of variation, specific surface area, and volume average pore diameter can be measured by, for example, laser diffraction/scattering particle size distribution measurement.

Further, the chromatographic carrier obtained as described above is useful for separating an antibody or a fragment thereof from a sample containing an antibody or a fragment thereof. Examples of the sample include blood components such as whole blood, serum, plasma, various blood cells, blood clots, and platelets; body fluids such as urine, semen, breast milk, sweat, interstitial fluid, interstitial lymph fluid, bone marrow fluid, tissue fluid, saliva, gastric fluid, synovial fluid, pleural effusion, bile, ascites, and amniotic fluid; bacterial cell fluids; culture media for cell culture; cell culture supernatants; and various liquid samples such as lysates of tissue cells.

### Examples

Hereinafter, the present invention will be described in more detail with reference to examples. However, the present invention is not limited only to these examples.

### (Preparation Example: Preparation of Ligands (Immunoglobulin-Binding Proteins))

Immunoglobulin-binding proteins PrA-0 to PrA-3 were obtained. PrA-0 is an immunoglobulin-binding protein including a homopentamer in which C domains of protein A (SEQ ID NO. 1) are serially linked. PrA-1 to PrA-3 are mutants in which mutations described in Table 1 are introduced into each immunoglobulin-binding domain of PrA-0.

**[Table 1]**

| Immunoglobulin-binding protein | Parent domain | Introduced mutations | Mutant domain | Number of domains |
|---|---|---|---|---|
| PrA-0 | SEQ ID No. 1 (Ala Asp Asn Lys Phe Asn Lys Glu Gln Gln Asn Ala Phe Tyr Glu Ile Leu His Leu Pro Asn Leu Thr Glu Glu Gln Arg Asn Gly Phe Ile Gln Ser Leu Lys Asp Asp Pro Ser Val Ser Lys Glu Ile Leu Ala Glu Ala Lys Lys Leu Asn Asp Ala Gln Ala Pro Lys) | - | SEQ ID No. 1 | 5 |
| PrA-1 | | A1V, G29A | SEQ ID No. 2 | 5 |
| PrA-2 | | A1V, N3A, N6D, N11Q, G29A, K49R | SEQ ID No. 3 | 5 |
| PrA-3 | | A1V, N11E, G29A | SEQ ID No. 4 | 5 |

Expression and purification of PrA-0 to PrA-3 were carried out as follows. Escherichia coli BL21(DE3) was transformed using plasmids encoding PrA-0 to PrA-3, and the obtained transformants were cultured in a rich medium at 37°C until the logarithmic growth phase. Thereafter, isopropyl-β-thiogalactopyranoside (manufactured by FUJIFILM Wako Pure Chemical Corporation) was added to the medium to a final concentration of 1 mM, and the culture was further continued at 37°C for 4 hours to express the target proteins. Subsequently, the culture solution was centrifuged to remove the supernatant, and the obtained cells were disrupted by adding a 30 mM Tris buffer at pH 9.5 containing lysozyme derived from egg white (manufactured by FUJIFILM Wako Pure Chemical Corporation) and polyoxyethylene (10) octylphenyl ether (manufactured by FUJIFILM Wako Pure Chemical Corporation). The recombinant immunoglobulin-binding proteins were purified from the obtained cell lysate by cation exchange chromatography (SP-Sepharose FF, manufactured by GE Healthcare Biosciences) and anion exchange chromatography (Q-Sepharose FF, manufactured by GE Healthcare Biosciences). The purified immunoglobulin-binding proteins were dialyzed against a 10 mM citrate buffer at pH 6.0. The purity of the recombinant immunoglobulin-binding protein confirmed by SDS-PAGE was 95% or more.

### [Example 1]

### (Process 1: Polymerization Process of Porous Particles)

2.69 g of polyvinyl alcohol (PVA-217, manufactured by Kuraray Corporation) was added to 448 g of pure water, and the mixture was heated and stirred to dissolve the polyvinyl alcohol, thereby obtaining an aqueous solution. On the other hand, a monomer composition consisting of 3.63 g of divinylbenzene (manufactured by FUJIFILM Wako Pure Chemical Corporation), 0.36 g of 1-ethyl-4-vinylbenzene (manufactured by ChemSampCo, Inc), and 14.15 g of glycidyl methacrylate (manufactured by MITSUBISHI GAS CHEMICAL COMPANY, INC.) was dissolved in 29.38 g of 2-octanone (manufactured by Toyo Gosei Co., Ltd) to prepare a monomer solution. Next, the entire amount of the aqueous solution was charged into a separable flask, and a thermometer, a stirring blade, and a condenser were attached thereto, followed by setting in a warm water bath and starting stirring under a nitrogen atmosphere. The entire amount of the monomer solution was added into the separable flask, and when the internal temperature reached 85°C by heating with the warm water bath, 1.34 g of 2,2'-azobis(methyl isobutyrate) (manufactured by FUJIFILM Wako Pure Chemical Corporation) was added, and the internal temperature was raised to 86°C. Thereafter, stirring was carried out for 3 hours while maintaining the temperature at 86°C. Subsequently, the reaction liquid was cooled, then filtered, and washed with pure water and ethanol. The washed particles were dispersed in pure water, and decantation was carried out 3 times to remove fine particles. Next, the particles were dispersed in pure water so that the concentration of the particles became 10 mass%, thereby obtaining a porous particle dispersion. The porous particles contained in this dispersion are referred to as "Porous Particles 1".

Subsequently, 0.956 g of adipic acid dihydrazide (manufactured by Tokyo Chemical Industry Co., Ltd.), 8 g of thioglycerol (manufactured by TOKYO CHEMICAL INDUSTRY CO., LTD.), and 1.418 g of diisopropylethylamine (manufactured by TOKYO CHEMICAL INDUSTRY CO., LTD.) were added to 100 g of the Porous Particles 1 dispersion, and the mixture was heated to 70°C and stirred for 8 hours while maintaining 70°C. Subsequently, the reaction liquid was cooled, then filtered, and washed with pure water and ethanol. Next, the particles were dispersed in pure water so that the concentration of the particles became 10 mass%, thereby obtaining a porous particle dispersion. The porous particles contained in this dispersion are referred to as "Porous Particles 2".

Next, ethylene glycol diglycidyl ether was reacted with hydroxyl groups derived from thioglycerol contained in Porous Particles 2. Specifically, 8.7 g of pure water, 1.2 g of sodium sulfate (manufactured by FUJIFILM Wako Pure Chemical Corporation), and 0.10 g of sodium carbonate (manufactured by FUJIFILM Wako Pure Chemical Corporation) were mixed to obtain a carbonate buffer (pH 11.2). To this carbonate buffer, 0.5 g of ethylene glycol diglycidyl ether (Denacol EX-810, manufactured by Nagase ChemteX Corporation) and 8 mL of Porous Particles 2 were added, followed by shaking and stirring at 23°C for 16 hours. Subsequently, the reaction liquid was filtered and washed with pure water, and then the particles were dispersed in pure water so that the concentration of the particles became 50 vol%, thereby obtaining a porous particle dispersion. The porous particles contained in this dispersion are referred to as "Porous Particles 3".

### (Process 2: Washing Process of Porous Particles)

Next, Porous Particles 3 were washed.

That is, a funnel (Kiriyama funnel, manufactured by Kiriyama Glass Works Co.) equipped with a filter (filter paper for Kiriyama funnel, manufactured by Kiriyama glass. CO., ltd.) was prepared, and 16 mL of the dispersion of Porous Particles 3 was introduced into the funnel under reduced pressure conditions. In this manner, pure water was separated from the dispersion by suction filtration, thereby allowing the solid content of the dispersion to settle at the bottom of the funnel (filter bed) to form a bed.

Thereafter, while maintaining suction under reduced pressure, 8 mL of a 0.5 M aqueous sodium hydroxide solution (pH 13.7) was added and passed through, followed by filtration without stirring so as to flow from the upper side to the lower side of the bed, thereby performing "cut-through washing (hereinafter also simply referred to as "S" or "washing S")" twice.

Next, with the tip of the funnel closed, 8 mL of a 0.5 M aqueous sodium hydroxide solution (pH 13.7) was added, the entire slurry in the funnel was stirred (10 minutes, 60 rpm), and then suction filtration was performed to carry out "reslurry washing (hereinafter also simply referred to as "R" or "washing R")" once.

Next, the particles were dispersed in pure water so that the concentration of the particles became 50 vol%, thereby obtaining a porous particle dispersion. The porous particles contained in this dispersion are referred to as "Porous Particles 4".

### (Process 3: Binding Process of Ligand)

Next, a ligand was bound to Porous Particles 4. That is, 28.8 g of pure water, 5.4 g of sodium sulfate (manufactured by FUJIFILM Wako Pure Chemical Corporation), 0.2 g of sodium hydrogen carbonate (manufactured by FUJIFILM Wako Pure Chemical Corporation), and 0.16 g of sodium carbonate (manufactured by FUJIFILM Wako Pure Chemical Corporation) were mixed to obtain a carbonate buffer (pH 9.3). To 25 mL of this carbonate buffer, 0.17 g of the immunoglobulin-binding protein PrA-1 (modified protein A that is a pentamer of the amino acid sequence domain of SEQ ID NO. 2) prepared in the Preparation Example and 8 mL of Porous Particles 4 were added, followed by shaking and stirring at 23°C for 1.5 hours, and the reaction liquid was filtered. Next, 8.8 g of pure water, 0.1 g of sodium sulfate (manufactured by FUJIFILM Wako Pure Chemical Corporation), and 0.03 g of sodium hydroxide (manufactured by FUJIFILM Wako Pure Chemical Corporation) were mixed to obtain a buffer, after which 4.5 g of thioglycerol (manufactured by TOKYO CHEMICAL INDUSTRY CO., LTD.) was added to prepare a hydrophilization reaction solution, and the hydrophilization reaction solution thus prepared was added to the porous particles to which a ligand had been bound, followed by shaking and stirring at 23°C for 16 hours to perform a hydrophilization reaction. Next, the particles were dispersed in pure water so that the concentration of the particles became 50 vol%, thereby obtaining a porous particle dispersion. The porous particles contained in this dispersion are referred to as "Porous Particles 5".

### (Process 4: Washing Process After Binding Process)

Next, Porous Particles 5 were washed.

That is, a funnel (Kiriyama funnel, manufactured by Kiriyama Glass Works Co.) equipped with a filter (filter paper for Kiriyama funnel, manufactured by Kiriyama glass. CO., ltd.) was prepared, and 16 mL of the dispersion of Porous Particles 5 was introduced into the funnel under reduced pressure conditions. In this manner, pure water was separated from the dispersion by suction filtration, thereby allowing the solid content of the dispersion to settle at the bottom of the funnel (filter bed) to form a bed.

Thereafter, while maintaining suction under reduced pressure, 8 mL of a 0.1 M aqueous sodium carbonate solution (pH 11.4) was added and passed through, thereby performing "washing S" twice.

Next, with the tip of the funnel closed, 8 mL of a 0.1 M aqueous sodium carbonate solution (pH 11.4) was added, the entire slurry in the funnel was stirred (10 minutes, 60 rpm), and then suction filtration was performed to carry out "washing R" once.

Subsequently, after neutralization with a sodium citrate buffer, the particles were dispersed in pure water so that the concentration of the particles became 50 vol%, thereby obtaining a porous particle dispersion. The porous particles contained in this dispersion are referred to as "Carrier 1".

### [Example 2]

Carrier 2 was obtained in the same manner as in Example 1, except that the 0.5 M aqueous sodium hydroxide solution used in "Process 2: Washing Process of Porous Particles" was changed to 0.1 M hydrochloric acid.

### [Example 3]

Carrier 3 was obtained in the same manner as in Example 1, except that the 0.5 M aqueous sodium hydroxide solution used in "Process 2: Washing Process of Porous Particles" was changed to 1.0 M hydrochloric acid.

### [Example 4]

Carrier 4 was obtained in the same manner as in Example 1, except that the 0.5 M aqueous sodium hydroxide solution used in "Process 2: Washing Process of Porous Particles" was changed to 0.01 M hydrochloric acid.

### [Example 5]

Carrier 5 was obtained in the same manner as in Example 1, except that the 0.5 M aqueous sodium hydroxide solution used in "Process 2: Washing Process of Porous Particles" was changed to a 0.1 M aqueous hydrogen peroxide solution.

### [Example 6]

Carrier 6 was obtained in the same manner as in Example 1, except that the 0.5 M aqueous sodium hydroxide solution used in "Process 2: Washing Process of Porous Particles" was changed to a 0.1 M aqueous peracetic acid solution.

### [Example 7]

Carrier 7 was obtained in the same manner as in Example 1, except that the 0.5 M aqueous sodium hydroxide solution used in "Process 2: Washing Process of Porous Particles" was changed to a 1.0 M aqueous sodium hydroxide solution.

### [Example 8]

Carrier 8 was obtained in the same manner as in Example 1, except that the 0.5 M aqueous sodium hydroxide solution used in "Process 2: Washing Process of Porous Particles" was changed to a 0.1 M aqueous sodium hydroxide solution.

### [Example 9]

Carrier 9 was obtained in the same manner as in Example 1, except that the number of times of "washing S" carried out in "Process 2: Washing Process of Porous Particles" was changed from 2 times to 4 times.

### [Example 10]

Carrier 10 was obtained in the same manner as in Example 1, except that the number of times of "washing S" carried out in "Process 2: Washing Process of Porous Particles" was changed from 2 times to 1 time.

### [Example 11]

Carrier 11 was obtained in the same manner as in Example 1, except that the 0.1 M aqueous sodium carbonate solution (pH 11.4) used in "Process 4: Washing Process After Binding Process" was changed to a 0.1 M aqueous sodium phosphate solution (prepared by mixing a 0.1 M aqueous sodium dihydrogen phosphate solution and a 0.1 M aqueous disodium hydrogen phosphate solution so as to have a pH of 7.5).

### [Example 12]

Carrier 12 was obtained in the same manner as in Example 1, except that the 0.1 M aqueous sodium carbonate solution (pH 11.4) used in "Process 4: Washing Process After Binding Process" was changed to a 0.5 M aqueous sodium phosphate solution (prepared by mixing a 0.5 M aqueous sodium dihydrogen phosphate solution and a 0.5 M aqueous disodium hydrogen phosphate solution so as to have a pH of 7.5).

### [Example 13]

Carrier 13 was obtained in the same manner as in Example 1, except that the 0.1 M aqueous sodium carbonate solution (pH 11.4) used in "Process 4: Washing Process After Binding Process" was changed to a 0.01 M aqueous sodium phosphate solution (prepared by mixing a 0.01 M aqueous sodium dihydrogen phosphate solution and a 0.01 M aqueous disodium hydrogen phosphate solution so as to have a pH of 7.5).

### [Example 14]

Carrier 14 was obtained in the same manner as in Example 1, except that the 0.1 M aqueous sodium carbonate solution (pH 11.4) used in "Process 4: Washing Process After Binding Process" was changed to a 0.1 M aqueous sodium carbonate solution (prepared by adding an aqueous sodium hydroxide solution so as to have a pH of 12.5).

### [Example 15]

Carrier 15 was obtained in the same manner as in Example 1, except that the 0.1 M aqueous sodium carbonate solution (pH 11.4) used in "Process 4: Washing Process After Binding Process" was changed to a 0.1 M aqueous sodium carbonate solution (prepared by mixing a 0.1 M aqueous sodium hydrogen carbonate solution and a 0.1 M aqueous sodium carbonate solution so as to have a pH of 10).

### [Example 16]

Carrier 16 was obtained in the same manner as in Example 1, except that the number of times of "washing S" carried out in "Process 4: Washing Process After Binding Process" was changed from 2 times to 4 times.

### [Example 17]

Carrier 17 was obtained in the same manner as in Example 1, except that the number of times of "washing S" carried out in "Process 4: Washing Process After Binding Process" was changed from 2 times to 1 time.

### [Example 18]

Carrier 18 was obtained in the same manner as in Example 1, except that Porous Particles 3 obtained in "Process 1: Polymerization Process of Porous Particles" were changed to agarose-based particles (WorkBeads 40 ACT, manufactured by Bio-Works).

### [Example 19]

Carrier 19 was obtained in the same manner as in Example 1, except that the immunoglobulin-binding protein PrA-1 used in "Process 3: Binding Process of Ligand" was changed to immunoglobulin-binding protein PrA-0.

### [Example 20]

Carrier 20 was obtained in the same manner as in Example 1, except that the immunoglobulin-binding protein PrA-1 used in "Process 3: Binding Process of Ligand" was changed to immunoglobulin-binding protein PrA-3.

### [Example 21]

Carrier 21 was obtained in the same manner as in Example 1, except that the immunoglobulin-binding protein PrA-1 used in "Process 3: Binding Process of Ligand" was changed to immunoglobulin-binding protein PrA-2.

### [Example 22]

Carrier 22 was obtained in the same manner as in Example 1, except that the immunoglobulin-binding protein PrA-1 used in "Process 3: Binding Process of Ligand" was changed to Pierce (registered trademark) Recombinant Protein L (21189 manufactured by Thermo Fisher Scientific Inc.).

### [Example 23]

Carrier 23 was obtained in the same manner as in Example 1, except that the immunoglobulin-binding protein PrA-1 used in "Process 3: Binding Process of Ligand" was changed to Pierce (registered trademark) Recombinant Protein G (21193 manufactured by Thermo Fisher Scientific Inc.).

### [Comparative Example 1]

A carrier of Comparative Example 1 was obtained in the same manner as in Example 1, except that "Process 2: Washing Process of Porous Particles" was not carried out and the 0.1 M aqueous sodium carbonate solution (pH 11.4) used in "Process 4: Washing Process After Binding Process" was changed to a 0.5 M aqueous sodium hydroxide solution (pH 13.7).

### [Comparative Example 2]

A carrier of Comparative Example 2 was obtained in the same manner as in Example 1, except that the 0.5 M aqueous sodium hydroxide solution used in "Process 2: Washing Process of Porous Particles" was changed to pure water, and the 0.1 M aqueous sodium carbonate solution (pH 11.4) used in "Process 4: Washing Process After Binding Process" was changed to a 0.5 M aqueous sodium hydroxide solution (pH 13.7).

### [Comparative Example 3]

A carrier of Comparative Example 3 was obtained in the same manner as in Example 1, except that the 0.5 M aqueous sodium hydroxide solution used in "Process 2: Washing Process of Porous Particles" was changed to a 0.1 M aqueous sodium carbonate solution (pH 11.4).

### [Comparative Example 4]

A carrier of Comparative Example 4 was obtained in the same manner as in Comparative Example 1, except that the number of times of "washing S" carried out in "Process 4: Washing Process After Binding Process" was changed from 2 times to 6 times, and the number of times of "washing R" was changed from 1 time to 0 times.

### [Comparative Example 5]

A carrier of Comparative Example 5 was obtained in the same manner as in Comparative Example 1, except that the number of times of "washing S" carried out in "Process 4: Washing Process After Binding Process" was changed from 2 times to 0 times, and the number of times of "washing R" was changed from 1 time to 3 times.

### [Comparative Example 6]

A carrier of Comparative Example 6 was obtained in the same manner as in Example 1, except that "Process 2: Washing Process of Porous Particles" was not carried out.

### [Comparative Example 7]

A carrier of Comparative Example 7 was obtained in the same manner as in Example 1, except that the 0.5 M aqueous sodium hydroxide solution used in "Process 2: Washing Process of Porous Particles" was changed to pure water.

### (Test Example 1) Dynamic Binding Capacity (DBC) Measurement Test

Using an AKTA avant 25 manufactured by Cytiva, the DBC of each carrier of Examples 1 to 21 and Comparative Examples with respect to a protein (human IgG antibody, 1875-0007 manufactured by LGC Limited) at a residence time of 4 minutes was measured. A column having a capacity of 4 mL (5 mm diameter × 200 mm length) was used as the column container, and as the protein, a solution prepared by dissolving the protein at 5 mg/mL in a 20 mM sodium phosphate/150 mM sodium chloride aqueous solution (pH 7.5) was used, and the DBC was calculated from the protein capture amount at 10% breakthrough of the elution front and the column packing volume, and was evaluated according to the following criteria. The results are shown in Tables 2 to 4 and 6.

### (Evaluation Criteria for DBC)

AA (Excellent): 62 mg/mL or more
A (Good): 61 mg/mL or more and less than 62 mg/mL
B (Poor): less than 61 mg/mL

### (Test Example 2) Protein Leakage Measurement Test

Using an AKTA avant 25 (manufactured by Cytiva), 7.5 mL of a cell culture solution (Herceptin, titer: 4.38 mg/mL) was loaded onto each carrier of the examples and comparative examples at a residence time of 4 minutes, and thereafter the antibody was recovered with an eluate. A column having a capacity of 0.8 mL (5 mm diameter × 40 mm length) was used as the column container, and a 100 mM aqueous sodium acetate solution (pH 3.3) was used as the eluate. Prior to elution, washing was carried out with a 20 mM sodium phosphate/500 mM sodium chloride aqueous solution (pH 7.5).

Next, for the carrier of Example 22, a Protein L-Ligand Leakage ELISA Kit (manufactured by Genaxxon bioscience GmbH) was used; for the carrier of Example 23, a Protein G ELISA Kit (manufactured by Alpha Diagnostic International) was used; and for the other carriers, a Protein A ELISA Kit (F740) was used. The protein amount in the eluate was determined from absorbance, and the antibody concentration in the eluate was also determined from absorbance, and based on these values, the amount of protein leakage (leach) per amount of antibody in the recovered eluate was calculated and evaluated according to the following criteria. The results are shown in Tables 2 to 6.

### (Evaluation Criteria for Protein Leakage)

AAA (Excellent): 8 ppm/IgG or less
AA (Very Good): more than 8 ppm/IgG and less than 10 ppm/IgG
A (Good): 10 ppm/IgG or more and less than 13 ppm/IgG
B (Poor): 13 ppm/IgG or more

### (Test Example 3) Aggregation Amount Evaluation

16 mL of the dispersion of each carrier of the Examples and Comparative Examples was passed through a wire mesh (manufactured by Taiyo. Co., Ltd., replacement mesh-type funnel, 30 mesh), and the particles remaining on the mesh were collected using pure water. The collected particle dispersion was transferred to an aluminum dish and heated on a hot plate at 200°C for 10 minutes to obtain dried particles. The weight of the dried particles was measured and defined as the aggregation amount (g). The smaller the obtained aggregation amount value, the lower the aggregation. The results are shown in Tables 2 to 6.

### (Test Example 4) Replacement Efficiency of Washing Liquid

The electrical conductivity of the washing liquid first used in "Process 4: Washing Process After Binding Process" was measured using a conductivity meter manufactured by HORIBA, Ltd. Subsequently, the final filtrate immediately before washing with a sodium citrate buffer carried out in "Process 4: Washing Process After Binding Process" was collected, and the electrical conductivity was measured in the same manner as described above. The replacement efficiency (%) of the washing liquid was calculated from the following equation. The closer this value is to 100%, the better the replacement efficiency. The results are shown in Tables 2 to 6. 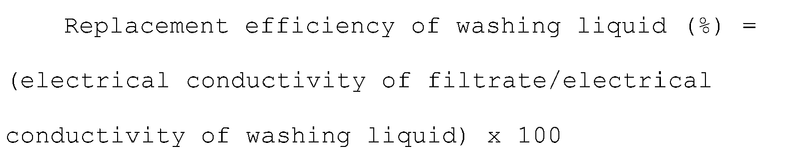 Replacement efficiency of washing liquid (%) = (electrical conductivity of filtrate/electrical conductivity of washing liquid) x 100

### (Test Example 5) Relative Hydrophilicity Evaluation

To 10 parts by mass of dried particles of Porous Particles 4 (particles before ligand binding) obtained in Processes 1 to 2 of each example and comparative example, 30 parts by mass of water was added, and the state of dispersion was visually confirmed.

Samples in which the particles were not aggregated and were cleanly dispersed were rated as "A", samples in which the particles were dispersed but small aggregates were observed were rated as "B", and samples in which relatively large aggregates remained were rated as "C". The results are shown in Tables 2 to 6.

**[Table 2]**

| | | Example 1 | Example 2 | Example 3 | Example 4 | Example 5 | Example 6 | Example 7 | Example 8 |
|---|---|---|---|---|---|---|---|---|---|
| Particle type | | Synthetic resin | | | | | | | |
| Ligand type | | PrA-1 | | | | | | | |
| Particle washing (before ligand binding) | Type of washing liquid | Sodium hydroxide | Hydrochloric acid | | | Hydrogen peroxide | Peracetic acid | Sodium hydroxide | |
| | Concentration | 0.5 M | 0.1 M | 1.0 M | 0.01 M | 0.1 M | 0.1 M | 1.0 M | 0.1 M |
| | pH | 13.7 | 1.0 | 0 | 2.0 | 6.3 | 4.6 | 14 | 13 |
| | Washing method | SSR | SSR | SSR | SSR | SSR | SSR | SSR | SSR |
| | Relative hydrophilicity | A | B | B | B | B | B | A | B |
| Carrier washing (after ligand binding) | Type of washing liquid | Sodium carbonate | | | | | | | |
| | Concentration | 0.1 M | | | | | | | |
| | pH | 11.4 | | | | | | | |
| | Washing method | SSR | | | | | | | |
| Protein leakage amount | Measured value (ppm/IgG) | 7.2 | 10.7 | 10.0 | 12.5 | 11.1 | 10.7 | 6.5 | 10.5 |
| | Evaluation | AAA | A | A | A | A | A | AAA | A |
| Aggregation amount evaluation (g) | | 0 | 0 | 0 | 0 | 0 | 0 | 0 | 0 |
| DBC | Measured value (mg/mL) | 62.8 | 62.0 | 62.1 | 62.5 | 62.2 | 62.0 | 62.4 | 62.7 |
| | Evaluation | AA | AA | AA | AA | AA | AA | AA | AA |
| Liquid replacement efficiency | | 101.1 | 101.9 | 99.8 | 98.1 | 100.6 | 99.4 | 98.3 | 101.0 |

**[Table 3]**

| | | Example 9 | Example 10 | Example 11 | Example 12 | Example 13 | Example 14 | Example 15 |
|---|---|---|---|---|---|---|---|---|
| Particle type | | Synthetic resin | | | | | | |
| Ligand type | | PrA-1 | | | | | | |
| Particle washing (before ligand binding) | Type of washing liquid | Sodium hydroxide | | | | | | |
| | Concentration | 0.5 M | | | | | | |
| | pH | 13.7 | | | | | | |
| | Washing method | SSSSR | SR | SSR | | | | |
| | Relative hydrophilicity | A | A | A | | | | |
| Carrier washing (after ligand binding) | Type of washing liquid | Sodium carbonate | | Sodium phosphate (NaH₂PO₄ + Na₂HPO₄) | | | Sodium carbonate + Sodium hydroxide | Sodium carbonate + Sodium hydrogen carbonate |
| | Concentration | 0.1 M | | 0.1 M | 0.5 M | 0.01 M | 0.1 M^{*1} | 0.1 M^{*1} |
| | pH | 11.4 | | 7.5 | 7.5 | 7.5 | 12.5 | 10.0 |
| | Washing method | SSR | | SSR | SSR | SSR | SSR | SSR |
| Protein leakage amount | Measured value (ppm/IgG) | 8.0 | 10.1 | 8.2 | 8.0 | 10.1 | 7.1 | 10.5 |
| | Evaluation | AAA | A | AA | AAA | A | AAA | A |
| Aggregation amount evaluation (g) | | 0.11 | 0 | 0 | 0 | 0 | 0 | 0 |
| DBC | Measured value (mg/mL) | 62.4 | 62.8 | 62.1 | 61.3 | 62.0 | 61.4 | 62.3 |
| | Evaluation | AA | AA | AA | A | AA | A | AA |
| Liquid replacement efficiency | | 98.2 | 99.8 | 101.9 | 100.6 | 99.4 | 98.6 | 101.3 |

| | | | | | | | | |
|---|---|---|---|---|---|---|---|---|
| *1: Sodium carbonate concentration | | | | | | | | |

**[Table 4]**

| | | Example 16 | Example 17 | Example 18 | Example 19 | Example 20 | Example 21 |
|---|---|---|---|---|---|---|---|
| Particle type | | Synthetic resin | | Agarose | Synthetic resin | | |
| Ligand type | | PrA-1 | | | PrA-0 | PrA-3 | PrA-2 |
| Particle washing (before ligand binding) | Type of washing liquid | Sodium hydroxide | | | | | |
| | Concentration | 0.5 M | | | | | |
| | pH | 13.7 | | | | | |
| | Washing method | SSR | | | | | |
| | Relative hydrophilicity | A | | | | | |
| Carrier washing (after ligand binding) | Type of washing liquid | Sodium carbonate | | | | | |
| | Concentration | 0.1 M | | | | | |
| | pH | 11.4 | | | | | |
| | Washing method | SSSSR | SR | SSR | | | |
| Protein leakage amount | Measured value (ppm/IgG) | 6.9 | 12.9 | 7.5 | 8.8 | 9.2 | 7.9 |
| | Evaluation | AAA | A | AAA | AA | AA | AAA |
| Aggregation amount evaluation (g) | | 0.14 | 0 | 0 | 0 | 0 | 0 |
| DBC | Measured value (mg/mL) | 62.5 | 62.0 | 62.3 | 61.0 | 62.2 | 62.4 |
| | Evaluation | AA | AA | AA | A | AA | AA |
| Liquid replacement efficiency | | 100.0 | 93.7 | 99.7 | 100.5 | 99.3 | 99.9 |

**[Table 5]**

| | | Example 22 | Example 23 |
|---|---|---|---|
| Particle type | | Synthetic resin | |
| Ligand type | | Protein L | Protein G |
| Particle washing (before ligand binding) | Type of washing liquid | Sodium hydroxide | |
| | Concentration | 0.5 M | |
| | pH | 13.7 | |
| | Washing method | SSR | |
| | Relative hydrophilicity | A | |
| Carrier washing (after ligand binding) | Type of washing liquid | Sodium carbonate | |
| | Concentration | 0.1 M | |
| | pH | 11.4 | |
| | Washing method | SSR | |
| Protein leakage amount | Measured value (ppm/IgG) | 8.5 | 7.7 |
| | Evaluation | AA | AAA |
| Aggregation amount evaluation (g) | | 0 | 0 |
| Liquid replacement efficiency | | 99.2 | 99.8 |

**[Table 6]**

| | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|
| Particle type | | Synthetic resin | | | | | | |
| Ligand type | | PrA-1 | | | | | | |
| Particle washing (before ligand binding) | Type of washing liquid | No washing | Pure water (pH 7) | Sodium carbonate | No washing | No washing | No washing | Pure water (pH 7) |
| | Concentration | | | 0.1 M | | | | |
| | pH | | | 11.4 | | | | |
| | Washing method | | SSR | SSR | | | | SSR |
| | Relative hydrophilicity | C | C | C | C | C | C | C |
| Carrier washing (after ligand binding) | Type of washing liquid | Sodium hydroxide | | Sodium carbonate | Sodium hydroxide | | Sodium carbonate | |
| | Concentration | 0.5 M | | 0.1 M | 0.5 M | | 0.1 M | |
| | pH | 13.7 | | 11.4 | 13.7 | | 11.4 | |
| | Washing method | SSR | | SSR | SSSSSS | RRR | SSR | |
| Protein leakage amount | Measured value (ppm/ IgG) | 11.1 | 11.0 | 14.2 | 10.6 | 14.2 | 14.9 | 14.8 |
| | Evaluation | A | A | B | A | B | B | B |
| Aggregation amount evaluation (g) | | 0 | 0 | 0 | 0.22 | 0 | 0 | 0 |
| DBC | Measured value (mg/mL) | 60.3 | 60.3 | 62.2 | 60.8 | 60.4 | 62.5 | 62.4 |
| | Evaluation | B | B | AA | B | B | AA | AA |
| Liquid replacement efficiency | | 101.1 | 101.0 | 99.6 | 101.1 | 94.6 | 100.3 | 100.2 |

## Claims

1. A production method for a chromatographic carrier, comprising the following solid-phase carrier washing process, ligand binding process, and ligand-bound carrier washing process:
(solid-phase carrier washing process) a process of washing a solid-phase carrier with at least one washing liquid selected from the group consisting of a washing liquid containing hydrogen peroxide, a washing liquid containing peracetic acid, and a washing liquid having a pH of 0 or more and 3 or less or a pH of more than 12.5 and 14 or less (excluding a washing liquid containing hydrogen peroxide and a washing liquid containing peracetic acid), thereby hydrophilizing a surface of the solid-phase carrier;
(ligand binding process) a process of binding a proteinaceous ligand to the solid-phase carrier washed in the solid-phase carrier washing process; and
(ligand-bound carrier washing process) a process of washing the carrier obtained in the ligand binding process.

2. The production method for a chromatographic carrier according to claim 1, wherein the ligand-bound carrier washing process is a process of washing with a washing liquid having a pH of more than 3 and 12.5 or less.

3. The production method for a chromatographic carrier according to claim 2, wherein the ligand-bound carrier washing process is a process of washing the carrier obtained in the ligand binding process two or more times in total with a washing liquid having a pH of more than 3 and 12.5 or less.

4. The production method for a chromatographic carrier according to claim 2, wherein the ligand-bound carrier washing process is a process of washing the carrier obtained in the ligand binding process 3 or 4 times in total with a washing liquid having a pH of more than 3 and 12.5 or less.

5. The production method for a chromatographic carrier according to any one of claims 1 to 4, wherein the ligand-bound carrier washing process is a process including the following ligand-bound carrier bed formation process, ligand-bound carrier liquid-passing washing process, and ligand-bound carrier stirring washing process:
(ligand-bound carrier bed formation process) a process of filling a ligand-bound carrier into a container to form a ligand-bound carrier bed;
(ligand-bound carrier liquid-passing washing process) a process of washing the ligand-bound carrier bed formed in the ligand-bound carrier bed formation process by passing a washing liquid having a pH of more than 3 and 12.5 or less therethrough one or more times; and
(ligand-bound carrier stirring washing process) a process of washing the ligand-bound carrier after the ligand-bound carrier liquid-passing washing process by stirring in a washing liquid having a pH of more than 3 and 12.5 or less one or more times.

6. The production method for a chromatographic carrier according to claim 5, wherein the number of times of liquid-passing washing in the ligand-bound carrier liquid-passing washing process is 2 to 5.

7. The production method for a chromatographic carrier according to any one of claims 1 to 6, wherein the solid-phase carrier washing process is a process of hydrophilizing a surface of the solid-phase carrier, the process including the following solid-phase carrier bed formation process and solid-phase carrier liquid-passing washing process:
(solid-phase carrier bed formation process) a process of filling a solid-phase carrier into a container to form a solid-phase carrier bed; and
(solid-phase carrier liquid-passing washing process) a process of washing the solid-phase carrier bed formed in the solid-phase carrier bed formation process by passing at least one washing liquid selected from the group consisting of a washing liquid containing hydrogen peroxide, a washing liquid containing peracetic acid, and a washing liquid having a pH of 0 or more to 3 or less or a pH of more than 12.5 and 14 or less (excluding a washing liquid containing hydrogen peroxide and a washing liquid containing peracetic acid) therethrough one or more times.

8. The production method for a chromatographic carrier according to any one of claims 1 to 6, wherein the solid-phase carrier washing process is a process of hydrophilizing a surface of the solid-phase carrier, the process including the following solid-phase carrier bed formation process, solid-phase carrier liquid-passing washing process, and solid-phase carrier stirring washing process:
(solid-phase carrier bed formation process) a process of filling a solid-phase carrier into a container to form a solid-phase carrier bed;
(solid-phase carrier liquid-passing washing process) a process of washing the solid-phase carrier bed formed in the solid-phase carrier bed formation process by passing at least one washing liquid selected from the group consisting of a washing liquid containing hydrogen peroxide, a washing liquid containing peracetic acid, and a washing liquid having a pH of 0 or more to 3 or less or a pH of more than 12.5 and 14 or less (excluding a washing liquid containing hydrogen peroxide and a washing liquid containing peracetic acid) therethrough one or more times; and
(solid-phase carrier stirring washing process) a process of washing the solid-phase carrier after the solid-phase carrier liquid-passing washing process by stirring in at least one washing liquid selected from the group consisting of a washing liquid containing hydrogen peroxide, a washing liquid containing peracetic acid, and a washing liquid having a pH of 0 or more and 3 or less or a pH of more than 12.5 and 14 or less (excluding a washing liquid containing hydrogen peroxide and a washing liquid containing peracetic acid) one or more times.

9. The production method for a chromatographic carrier according to claim 7 or 8, wherein the number of times of liquid-passing washing in the solid-phase carrier liquid-passing washing process is 2 to 5.

10. The production method for a chromatographic carrier according to claim 7 or 8, wherein the sum of the number of times of liquid-passing washing of the solid-phase carrier liquid-passing washing process and the number of times of liquid-passing washing of the ligand-bound carrier liquid-passing washing process is 2 to 8.

11. The production method for a chromatographic carrier according to any one of claims 1 to 10, wherein the ligand-bound carrier washing process is a process of washing with a washing liquid having a pH of 10.5 or more and 12 or less.

12. The production method for a chromatographic carrier according to any one of claims 1 to 11, wherein the washing liquid used in the solid-phase carrier washing process contains a strongly acidic pH adjusting agent or a strongly basic pH adjusting agent, and the washing liquid used in the ligand-bound carrier washing process contains an acidic pH adjusting agent or a basic pH adjusting agent.

13. The production method for a chromatographic carrier according to any one of claims 1 to 12, wherein the washing liquid used in the solid-phase carrier washing process is a washing liquid having a pH of 13.2 or more and 14 or less (excluding a washing liquid containing hydrogen peroxide and a washing liquid containing peracetic acid).

14. The production method for a chromatographic carrier according to any one of claims 1 to 13, wherein the solid-phase carrier washing process is a process of washing a solid-phase carrier two or more times in total with at least one washing liquid selected from the group consisting of a washing liquid containing hydrogen peroxide, a washing liquid containing peracetic acid, and a washing liquid having a pH of 0 or more and 3 or less or a pH of more than 12.5 and 14 or less (excluding a washing liquid containing hydrogen peroxide and a washing liquid containing peracetic acid).

15. The production method for a chromatographic carrier according to any one of claims 1 to 14, wherein the solid-phase carrier washing process is a process of washing a solid-phase carrier 3 to 4 times in total with at least one washing liquid selected from the group consisting of a washing liquid containing hydrogen peroxide, a washing liquid containing peracetic acid, and a washing liquid having a pH of 0 or more and 3 or less or a pH of more than 12.5 and 14 or less (excluding a washing liquid containing hydrogen peroxide and a washing liquid containing peracetic acid).

16. The production method for a chromatographic carrier according to any one of claims 1 to 15, wherein the proteinaceous ligand is a modified protein A.
